# FASCICULE DE BREVET EUROPEEN

(11) **EP 1 669 060 B1**
(45) Date de publication et mention de la délivrance du brevet: **07.05.2008**
(21) Numéro de dépôt: 05300982.5
(22) Date de dépôt: 30.11.2005
(51) Int. Cl.: A61K 8/44, A61Q 19/00

(54) **Nouveaux dérivés sulfamides et leur utilisation cosmétique**
Neue Sulfamid-Derivate und deren kosmetische Verwendung
New sulfamide derivatives and their cosmetic use

(30) Priorité: 30.11.2004 FR 0452821
(43) Date de publication de la demande: 14.06.2006
(73) Titulaire: L'ORÉAL, 75008 Paris (FR)
(72) Inventeur: Marat, Xavier, 75011 Paris (FR); Muller, M. Benoît, 75010 Paris (FR)
(74) Mandataire: Le Coupanec, Pascale A.M.P.

(56) Documents cités:
- EP-A- 0 549 079
- EP-A- 1 544 208
- WO-A-03/029226
- WO-A-03/097589
- WO-A-20/04050640
- FR-A- 2 355 896
- LODEN M ET AL: "A DOUBLE-BLIND STUDY COMPARING THE EFFECT OF GLYCERIN AND UREA ON DRY, ECZEMATOUS SKIN IN ATOPIC PATIENTS" ACTA DERMATO-VENEREOLOGICA, vol. 82, no. 1, 2002, pages 45-47, XP001121921 ISSN: 0001-5555
- DATABASE CAPLUS CHEMICAL ABSTRACTS SERVICE, COLUMBUS, OHIO, US; 1953, AZIENDE CHIMICHE PRODOTTI GEMELLO: "Bactericide composition for animals, particularly chickens" XP002371016 Database accession no. 1953:23582 & IT 463 449 A 1951
- DATABASE CA [Online] CHEMICAL ABSTRACTS SERVICE, COLUMBUS, OHIO, US; 2004, ALCARAZ, LILIAN ET AL: "Novel N-aryl and N-heteroaryl sulfamide synthesis via palladium cross-coupling" XP002336739 extrait de STN Database accession no. 2004:553620 & ORGANIC LETTERS , 6(16), 2705-2708 CODEN: ORLEF7; ISSN: 1523-7060, 7 octobre 2004 (2004-10-07),
- DATABASE CA [Online] CHEMICAL ABSTRACTS SERVICE, COLUMBUS, OHIO, US; 2004, YONEKUBO, SHIGERU ET AL: "Human SGLT1 inhibitors containing benzylphenyl glucopyranoside or galactopyranoside derivatives" XP002336740 extrait de STN Database accession no. 2004:568609 & JP 2004 196788 A2 (KISSEI PHARMACEUTICAL CO., LTD., JAPAN) 15 juillet 2004 (2004-07-15)
- PATENT ABSTRACTS OF JAPAN vol. 1995, no. 07, 31 août 1995 (1995-08-31) & JP 07 097371 A (SHIONOGI & CO LTD), 11 avril 1995 (1995-04-11)
- G.DEWYNTER ET.AL.: "Sulfonyl Bis-N-Oxazolidinone (SB): A New Versatile Dielectrophile with Sequential Reactivity" TETRAHEDRON LETTERS, vol. 38, no. 50, 1997, pages 8691-8694, XP004097152
- J.M.DOUGHERTY ET.AL.: "Ring-Closing Metathesis Strategies to Cyclic Sulfamide Peptidomimetics" TETRAHEDRON, vol. 56, 2000, pages 9781-9790, XP004221946
- DATABASE CA [Online] CHEMICAL ABSTRACTS SERVICE, COLUMBUS, OHIO, US; 2002, MCREYNOLDS, MATTHEW D. ET AL: "A Concise Route to Structurally Diverse DMP 323 Analogues via Highly Functionalized 1,4-Diamines" XP002336741 extrait de STN Database accession no. 2002:917723 & ORGANIC LETTERS , 4(26), 4673-4676 CODEN: ORLEF7; ISSN: 1523-7060, 2002,

## Description

La présente invention concerne l'utilisation du sulfamide et de ses dérivés dans le domaine du soin de la peau et notamment à titre d'agent hydratant de la peau, des dérivés nouveaux du sulfamide ainsi que les compositions cosmétiques les contenant.

Le *Stratum Corneum* qui forme l'interface avec l'environnement externe desséchant, a notamment pour fonction de retarder la perte excessive d'eau provenant des couches plus profondes de l'épiderme. Le *Stratum Corneum* protège également contre les agressions mécaniques et le passage de produits chimiques et de micro-organismes étrangers. Il constitue également la première défense contre les rayonnements UV.

Le *Stratum Corneum,* d'une épaisseur de 10 µm, se compose de coméocytes empilés verticalement et entourés par une matrice de membranes enrichies en lipides. Ainsi, c'est un système à deux compartiments qui peut être comparé à un mur de briques, composé de cellules anucléées (les « briques ») et de membranes lamellaires intercellulaires (le « ciment »).

L'urée est un des ingrédients largement utilisés dans les formules hydratantes. Cependant elle peut modifier fortement la barrière cutanée en augmentant la Perte Insensible en Eau (PIE), ce qui diminue significativement la fonction barrière du *Stratum Corneum.*

Le glycérol, autre actif de référence dans ce domaine, possède l'inconvénient de rendre les formules collantes lorsqu'il est employé à haute concentration.

Il existe donc un besoin de trouver des solutions alternatives dans le domaine de l'hydratation de la peau.

Les inventeurs ont découvert que certains dérivés du sulfamide de formule générale (I) décrits ci-après sont de bons agents hydratants et ont un effet bénéfique en terme d'élasticité du *Stratum Corneum.*

Ainsi, la présente invention a pour objet l'utilisation cosmétique des composés de formule générale (I) : 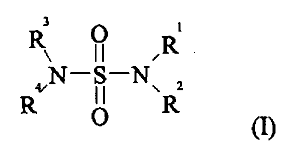 dans laquelle
- R¹, R², R³, R⁴ représentent indépendamment les uns des autres
   - un atome d'hydrogène,
   - un groupe (C₁-C₁₀)alkyle, de préférence un groupe (C₁-C₆)alkyle et mieux un groupe (C₁-C₄)alkyle, éventuellement substitué par 1 à 5 groupes choisis parmi -OR⁵, -NR⁶R⁷ et, -SiR⁸R⁹R¹⁰, où éventuellement 1 à 3 atomes de carbone dudit groupe (C₁-C₁₀)alkyle peuvent être remplacé(s), indépendamment les uns des autres, par un hétéroatome choisi parmi un atome d'azote, un atome de soufre, un atome d'oxygène, un atome de silicium et un groupe sulfonyle (-SO₂-) incluant par exemple le groupement -O-SO₂-NR⁶R⁷, ou bien
- (R¹ et R²) et/ou (R³ et R⁴) peuvent former, avec l'azote qui les porte, un hétérocycle de 5 à 7 chaînons éventuellement substitué par 1 à 4 groupes hydroxyle et/ou un groupe -NR⁶R⁷, l'hétérocycle étant choisi notamment parmi la pyrrolidine, la pipérazine, la morpholine, l'azépane, la pyrazolidine, l'imidazolidine, l'oxazolidine, ainsi que leurs homologues partiellement insaturés par exemple dérivés du pyrrole, la pyridine ou la pyrimidine, ou bien
- (R¹ et R³) ou (R² et R⁴) peuvent former, avec le groupe -N-(SO₂)-N- qui les porte, un hétérocycle de 5 à 9 chaînons éventuellement substitué par 1 à 4 groupes hydroxyle(s) et/ou un groupe NR⁶R⁷ et notamment choisi parmi les 1,1-dioxydes de 1,2,5-thiadiazolidine, de 1,2,6- thiadiazinane, de 1,2,7- thiadiazépane, de 1,2,8- thiadiazocane et de 1,2,9- thiadiazonane,
- R⁵, R⁶, R⁷ représentent, indépendamment les uns des autres,
   - un atome d'hydrogène, ou
   - un groupe (C₁ -C₆)alkyle, et
- R⁵ peut encore représenter un groupe tel que -OR⁵ représente un groupe phosphate, ou sulfate,
- R⁸, R⁹, R¹⁰ représentent indépendamment les uns des autres un groupe (C₁-C₆)alkyle,
ainsi que leurs sels cosmétiquement acceptables, leurs solvates tels que les hydrates et leurs isomères, pour le soin non thérapeutique de la peau, et particulièrement à titre d'agent hydratant, notamment à un pH non alcalin.

La présente invention a en outre pour objet une composition cosmétique comprenant un composé de formule (I), dans un milieu physiologiquement acceptable, notamment à un pH non alcalin.

De façon préférée, la présente invention a également pour objet une composition cosmétique comprenant un composé de formule (I) tel que décrit ci-dessus, à l'exclusion du N,N'-bis(2-hydroxyéthyl)sulfamide, dans un milieu physiologiquement acceptable, notamment à un pH non alcalin.

La présente invention a par ailleurs pour objet une composition cosmétique comprenant, dans un milieu physiologiquement acceptable, notamment à un pH non alcalin, un composé de formule (I) tel que décrit ci-dessus et comprenant en outre au moins un additif choisi parmi une huile, un corps gras, un gélifiant, une charge, un filtre UV, un absorbeur d'odeur ou une matière colorante.

La présente invention a enfin pour objet un procédé de traitement cosmétique pour le soin non thérapeutique et/ou de maquillage de la peau caractérisé en ce qu'il comprend l'application sur la peau d'au moins une composition cosmétique selon la présente invention comprenant un composé de formule (I) tel que défini ci-dessus.

### Les composés de formule générale (I)

Dans le cadre de la présente invention, on préfère l'utilisation cosmétique des composés de formule générale (I)
dans laquelle
- R¹, R², R³, R⁴ représentent indépendamment les uns des autres
   - un atome d'hydrogène,
   - un groupe (2,2-diméthyldioxolane)méthyle,
   - un groupe (C₁-C₁₀)alkyte éventuellement substitué par 1 à 5 groupes choisis parmi -OR⁵, -NR⁶R⁷, -SiR⁸R⁹R¹⁰, ou bien
- (R¹ et R³) ou (R² et R⁴) forment ensemble, avec le groupe -N-(SO₂)-N- qui les porte, un hétérocycle de 6 à 8 chaînons choisi parmi les 1,1-dioxydes de 1,2,6-thiadiazinane, de 1,2,7-thiadiazépane, de 1,2,8-thiadiazonane, éventuellement substitués par 1 à 3 groupes hydroxyle,
- R⁵ représente
   - un atome d'hydrogène,
   - un groupe (C₁-C₄)alkyle,
   - un groupe -SO₂NRR', ou
- R⁶, R⁷ représentent indépendamment les uns des autres
   - un atome d'hydrogène, ou
   - un groupe (C₁-C₄)alkyle,
- R⁸, R⁹, R¹⁰ représentent indépendamment les uns des autres un groupe (C₁-C₄)alkyle, et
- R et R' représentent'indépendamment l'un de l'autre un atome d'hydrogène ou un groupe (C₁-C₄) alkyle,
ainsi que leurs sels cosmétiquement acceptables, leurs solvates tels que les hydrates et leurs isomères, pour le soin non thérapeutique de la peau, et particulièrement à titre d'agent d'hydratation, notamment à un pH non alcalin.

Ces composés de formule (I) seront par la suite nommés « composés préférés », pour des raisons de simplification.

On préfère tout particulièrement l'utilisation cosmétique des composés de formule générale (I)
dans laquelle
- R¹, R², R³, R⁴ représentent indépendamment les uns des autres :
   - un atome d'hydrogène,
   - un groupe (C₁-C₆)alkyle éventuellement substitué par 1 à 5 groupes hydroxyle, 1 à 2 groupes -SiMe₃ ou par un groupe -O-SO₂NRR', ou bien
- (R¹ et R³) représentent un atome d'hydrogène et (R² et R⁴) forment ensemble, avec le groupe -N-(SO₂)-N- qui les porte, un groupe 1,1-dioxyde de 1,2,7-thiadiazepane éventuellement substitué par 1 ou 2 groupes hydroxyle ou un groupe 1,1-dioxyde de 1,2,6-thiadiazinane éventuellement substitué par 1 ou 2 groupes hydroxyle,
- R et R' représentent indépendamment l'un de l'autre
   - un atome d'hydrogène, ou
   - un groupe (C₁-C₄)alkyle,
   ainsi que leurs sels cosmétiquement acceptables, leurs solvates tels que les hydrates et leurs isomères, pour le soin non thérapeutique de la peau, et particulièrement à titre d'agent d'hydratation, notamment à un pH non alcalin.
   Ces composés de formule (1) seront par la suite nommés « composés tout particulièrement préférés », pour des raisons de simplification.
   Le terme alkyle, dans le cadre de la présente invention, signifie une chaîne hydrocarbonée linéaire ou ramifiée, saturée ou insaturée, cyclique ou non cyclique. Parmi les groupes alkyle convenant à la mise en oeuvre de l'invention, on peut notamment citer le groupe méthyle, éthyle, isopropyle, n-propyle, n-butyle, t-butyle, -CH₂-t-butyle, pentyle, n-hexyle, cyclopropyle, cyclopentyle, cyclohexyle, cyclohexylméthyle, heptyle, octyle, nonyle, décyle, norbornyle et adamantyle.
   Parmi les groupes (C₁-C₁₀)alkyle dans lequel 1 à 3 atomes de carbone pouvant être remplacés par un hétéroatome, on peut citer le groupe (2,2-diméthyldioxolane)-méthyle.
   Par « pH non alcalin » on entend, dans le cadre de la présente invention, un pH compris entre 4 et 7, plus particulièrement entre 5 et 6.
   Certains des composés de formule (I) sont connus. Les documents suivants en décrivent certains et donnent des indications utiles aux modes de synthèse décris ci-après.
- L. F. Audrieth, M. Sveda, H. Sisler, M. Josetta Butler, Chemical Review, 1940, 26, 49-94,
- Paquin, Angewante Chemie, 1948, 60, 11/12, 316-320,
- K. Sprott, P. Hanson, Journal of Organic Chemistry, 2000, 65, 7913-7918,
- M. McReynolds, K. Sprott, P. Hanson, Organic Letters, 2002, 4, 26, 4673-4676,
- H. Preuschhof, H-U. Heyne, Organic Syntheses, Collective volume 6,78, étape 1,
- J.M. Dougherty, D.A. Probst, R.E. Robinson, J.D. Moore, T.A. Klein, K.A.Snelgrove, P.R. Hanson, Tetrahedron, 2000, 56, 9781-9790,
- G. Dewynter, M. Abdaoui, L. Toupet, J-L. Montero, Tetrahedron Letters, 1997,38, 8691-8694, et
- Y. Masui, H. Watanabe, T. Masui, Tetrahedron Letters, 2004, 45, 1853-1856, G. M. Atkins, E. M. Burgess, Journal of the American Chemical Society, 1968, 90, 4744-4745.

### Composés selon l'invention et leur mode de préparation

Les composés de formule (II), (III) et (IV) définis ci-après font également partie de l'invention.

Selon un aspect, l'invention a ainsi pour objet les composés de formule (II) 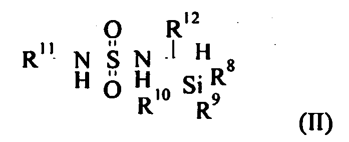 dans laquelle:
- R¹² représente un atome d'hydrogène, un groupe -SiR⁸R⁹R¹⁰, un groupe (C₁-C₉)alkyle éventuellement substitués par 1 à 5 groupes choisis parmi -OR⁵, -NR⁶R⁷,
- SiR⁸R⁹R¹⁰, où éventuellement 1 à 3 atomes de carbone dudit groupe (C₁-C₉)alkyle peuvent être remplacé(s), indépendamment les uns des autres, par un hétéroatome choisi parmi un atome d'azote, un atome de soufre, un atome d'oxygène, un atome de silicium ou un groupe-sulfonyle (-SO₂-),
- R¹¹ représente un atome d'hydrogène, un groupe (C₁-C₆)alkyle éventuellement substitué par 1 à 5 groupes choisis parmi -OR⁵ et/ou -SiR⁸R⁹R¹⁰ ou éventuellement par un groupe -OSO₂-NRR',
- R⁵, R⁶, R⁷, R⁸, R⁹, R¹⁰, R et R' étant tels que définis précédemment pour les composés préférés et tout particulièrement préférés de formule (I) ainsi que leurs solvates tels que les hydrates, leurs sels et leurs isomères.

On préfère les composés de formule (II) dans laquelle :
- R¹² représente un atome d'hydrogène, un groupe -SiMe₃ ou un groupe (C₁-C₆)alkyle éventuellement substitué par 1 à 5 groupes hydroxyle(s), 1 à 2 groupes -SiMe₃ ou par un groupe -O-SO₂NH₂,
- R¹¹ représente un atome d'hydrogène ou un groupe (C₁-C₆)alkyle éventuellement substitué par 1 à 2 groupes choisis parmi -OR⁵ et -SiMe₃,
- R⁵, R⁸, R⁹ et R¹⁰ étant tels que définis précédemment pour les composés préférés et tout particulièrement préférés de formule (I), ainsi que leurs solvates tels que les hydrates, leurs sels et leurs isomères.

Parmi les composés de formule (II) particulièrement préférés dans le cadre de la présente invention on peut notamment citer ceux pour lesquels
- R¹² représente un atome d'hydrogène, un groupe -SiMe₃ ou un groupe (C₁-C₆)alkyle éventuellement substitué par un groupe hydroxyle,
- R¹¹ représente un atome d'hydrogène ou un groupe (C₁-C₆)alkyle éventuellement substitué par 1 à 2 groupes hydroxyle, et
- R⁸, R⁹ et R¹⁰ représentent chacun un groupe méthyle, ainsi que leurs solvates tels que les hydrates, leurs sels et leurs isomères.

Selon un autre aspect, l'invention a aussi pour objet les composés de formule (III) 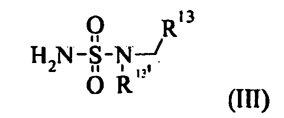 dans laquelle :
- R¹³ représente un (C₁-C₉)alkyle substitué par 1 à 5 groupes choisis parmi -OR⁵ et -NR⁶R⁷, ou un groupe -O-SO₂NRR',
- R^{13'} représente un atome d'hydrogène ou un groupe (C₁-C₄)alkyle, et
- R⁵, R⁶ et R⁷ sont tels que définis précédemment pour les composés préférés ou tout particulièrement préférés de formule (I),
- R, R' étant tels que définis précédemment pour les composés préférés et tout particulièrement préférés de formule (I),
- ainsi que leurs solvates tels que les hydrates, leurs sels et leurs isomères.

L'invention a plus particulièrement pour objet les composés de formule (III) dans laquelle R¹³) représente un (C₁-C₉) alkyle substitué par 1 à 5 groupes choisis parmi -OR⁵ ou un groupe -O-SO₂NRR', R⁵ représentant un atome d'hydrogène, un groupe (C₂-C₄) alkyle, un groupe -SO₂NRR', ou un groupe -SiR⁸R⁹R¹⁰ avec R⁸, R⁹, R¹⁰, R et R' étant tels que définis précédemment pour les composés préférés et tout particulièrement préférés de formule (I).

On préfère les composés de formule (III) dans laquelle :
- R¹³ représente un groupe (C₁-C₉)alkyle substitué par 1 à 5 groupe(s) hydroxyle(s) ou par un groupe -O-SO₂NH₂, et
- R^{13'} représente un atome d'hydrogène ou un groupe méthyle, ainsi que leurs solvates tels que les hydrates, leurs sels et leurs isomères.

Selon encore un autre aspect, l'invention a enfin pour objet les composés de formule (IV) 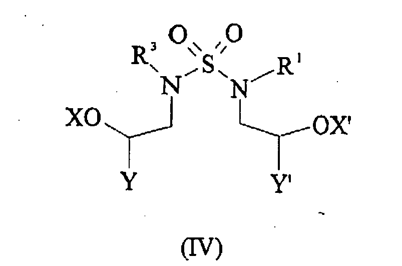
- X et X' prennent, indépendamment l'un de l'autre, la signification de R⁵ tel que précédemment défini pour les composés de formule (I) et Y et Y' représentent, indépendamment l'un de l'autre, un atome d'hydrogène, un groupe -CH₂Z, où Z représente un groupe amine ou un groupe -NR⁶R⁷ ou -OR⁵, ou bien
- au moins l'un des groupes 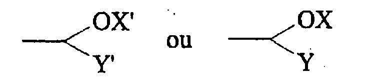 forme, indépendamment l'un de l'autre, un groupe diméthyldioxolane, ou bien :
- X et X' sont des atomes d'hydrogène et Y et Y' forment ensemble une liaison covalente,
- R¹, R³, R⁵, R⁶ et R⁷ prennent la même signification que celle définie pour les composés de formule (I), avantageusement, X, X', Y et Y' ne représentent pas simultanément un atome d'hydrogène.

L'invention a plus particulièrement pour objet les composés de formule (IV) dans laquelle Y et Y' ne sont pas simultanément un atome d'hydrogène lorsque X et X' représentent simultanément un groupe -CH₃.

On préfère les composés de formule (IV) dans laquelle :
- X et X' sont des atomes d'hydrogène et Y et Y' représentent un groupe -CH₂OR⁵, ou bien
- au mois l'un des groupes 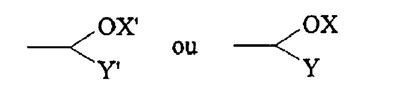 forme, un groupe diméthyldioxolane, ou bien
- X et X' sont des atomes d'hydrogène et Y et Y' forment ensemble une liaison covalente, et
- R¹, R³ et R⁵ prennent la même signification que celle définie par les composés de formule (I).
   Parmi les composés de formule (IV) particulièrement préférés dans le cadre de la présente invention on peut notamment citer ceux pour lesquels
- X et X' sont des atomes d'hydrogène et Y et Y' représentent un groupe - CH₂OH ou bien
- X et X' sont des atomes d'hydrogène et Y et Y' forment ensemble une liaison covalente, et
- R¹ et R³ représentent un atome d'hydrogène ou un groupe (C₁-C₆) alkyle.

Les sels acceptables pour l'usage non thérapeutique des composés décrits dans la présente invention comprennent des sels non toxiques conventionnels desdits composés tels que ceux formés à partir d'acides organiques ou inorganiques. A titre d'exemple, on peut citer les sels d'acides minéraux, tels que l'acide sulfurique, l'acide chlorhydrique, l'acide bromhydrique, l'acide iodhydrique, l'acide phosphorique, l'acide borique. On peut également citer les sels d'acides organiques, qui peuvent comporter un ou plusieurs groupes acide carboxylique, sulfonique, ou phosphonique. Il peut s'agir d'acides aliphatiques linéaires, ramifiés ou cycliques ou encore d'acides aromatiques. Ces acides peuvent comporter, en outre, un ou plusieurs hétéroatomes choisis parmi O et N, par exemple sous la forme de groupes hydroxyle. On peut notamment citer l'acide propionique, l'acide acétique, l'acide téréphtalique, l'acide citrique et l'acide tartrique.

Lorsque le composé de formule (I) comporte un groupe acide, la neutralisation du ou des groupes acides peut être effectuée par une base minérale, telle que LiOH, NaOH, KOH, Ca(OH)₂, NH₄OH, Mg(OH)₂ ou Zn(OH)₂; ou par une base organique telle qu'une alkylamine primaire, secondaire ou tertiaire, par exemple la triéthylamine ou la butylamine. Cette alkylamine primaire, secondaire ou tertiaire peut comporter un ou plusieurs atomes d'azote et/ou d'oxygène et peut donc comporter par exemple une ou plusieurs fonctions alcool; on peut notamment citer l'amino-2-méthyl-2-propanol, la triéthanolamine, la dimethylamino-2-propanol, le 2-amino-2-(hydroxymethyl)-1,3-propanediol. On peut encore citer la lysine ou la 3-(diméthylamino)propylamine.

Les solvates acceptables pour l'usage non thérapeutique des composés décrits dans la présente invention comprennent des solvates conventionnels tels que ceux formés lors de la dernière étape de préparation desdits composés du fait de la présence de solvants. A titre d'exemple, on peut citer les solvates dus à la présence d'eau ou d'alcools linéaires ou ramifiés comme l'éthanol ou l'isopropanol.

Les composés de formule (I) peuvent être préparés selon des techniques bien connues de l'homme de l'art, notamment selon un schéma 1 décrit ci-après.

Les modes d'obtention classiques du sulfamide sont réalisés à partir du chlorure de sulfuryle.

Les amines (ou diamines) choisies pour conduire aux composés de formule (I) peuvent être mises en réaction (de manière séquentielles pour les composés dissymétriques) avec une source électrophile de SO_{2,} telle que le sulfamide H₂NSO₂NH₂, le chlorure de sulfuryle SO₂Cl₂, ou la sulfonyl-bis 2-oxazolidine dans un solvant approprié (par exemple, respectivement l'acétonitrile, la pyridine, le diméthylformamide pour le sulfamide ou par exemple le dichlorométhane, le tétrahydrofurane pour le chlorure de sulfuryle) par exemple à une température variant de 0 à 140°C en présence ou non d'une base comme par exemple le diazabicyc1oundécène, la triéthylamine, ou la pyridine.

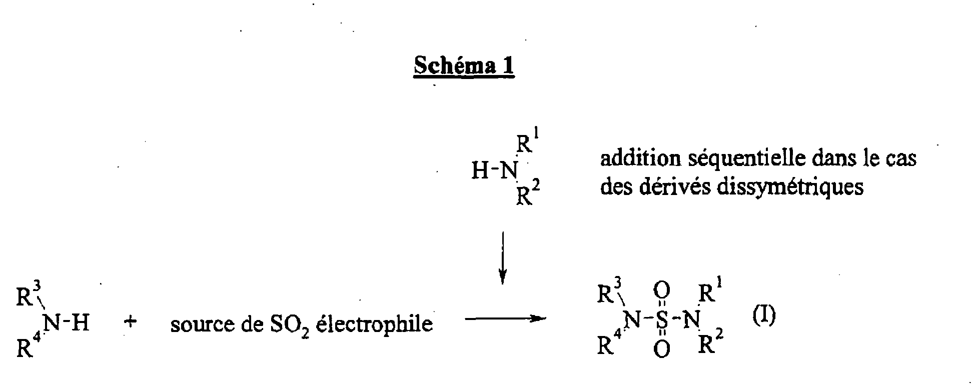

Les méthodes de synthèses des composés nouveaux respectivement de formules (II), (III) et (IV) sont détaillées ci-après.

Les composés de formule (II) peuvent être préparés selon le schéma 2 qui suit :

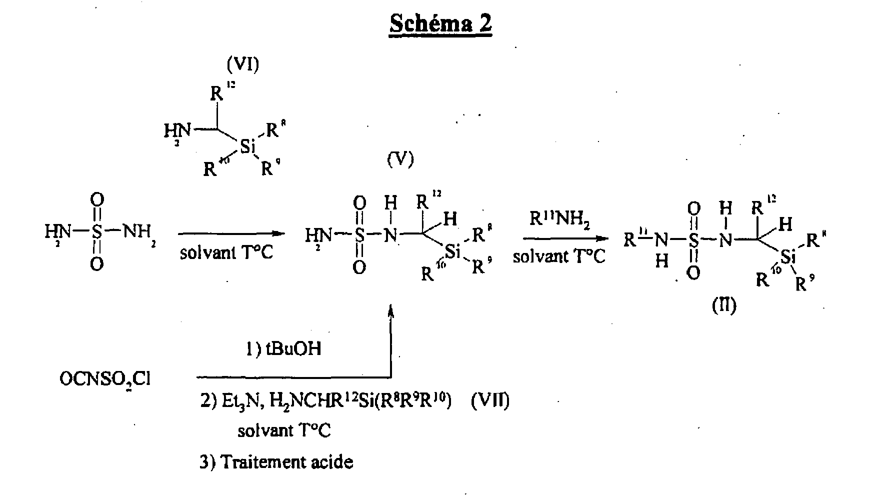

Les composés (II) peuvent être obtenus de deux manières :
- soit à partir du sulfamide en faisant réagir une mole d'amine silylée de formule (VI) par exemple dans un solvant polaire tel que l'acétonitrile, le diméthylformamide, ou la pyridine, par exemple à une température comprise entre 0 et 140 °C en présence ou non d'une quantité catalytique de base comme le diazabicycloundécène, pour obtenir les composés de formule (V), suivi d'une seconde addition d'une mole d'amine R¹¹NH₂ par exemple dans des conditions similaires,
- a soit à partir de l'isocyanate de chlorosulfonyle en effectuant une addition séquentielle de tertio-butanol et d'amine silylée de formule (VII), par exemple, dans un solvant tel que le dichlorométhane ou l'acétate d'éthyle, par exemple entre 0 et 20 °C en présence d'une base comme la triéthylamine ou la pyridine afin d'obtenir une sulfamide disymétrique dont le groupement NH₂, protégé sous forme de tertio-butoxycarbonyle, est libéré par un traitement acide avec par exemple de l'acide chlorhydrique ou de l'acide trifluoroacétique ; le composé (II) peut ensuite être formé par addition d'une mole d'amine de formule R¹¹NH₂ dans un solvant polaire tel que l'acétonitrile, le diméthylformamide ou la pyridine, par exemple à une température comprise entre 0 et 140 °C et en présence ou non d'une quantité catalytique de base comme par exemple le diazabicycloundécène.

Les composés de formule (III) peuvent être préparés selon le schéma 3 qui suit :

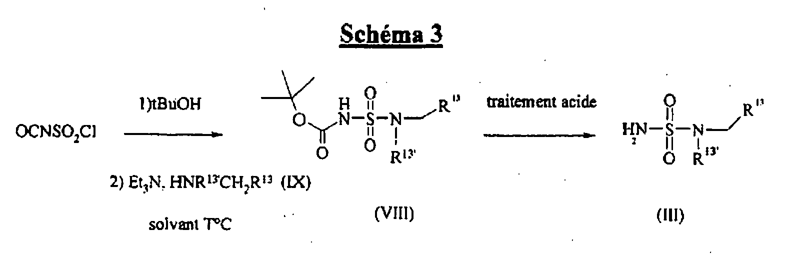

On fait réagir sur de l'isocyanate de chlorosulfonyle de manière séquentielle, une mole de tertio-butanol suivi d'une mole d'amine de formule (IX) en présence d'une base telle que la triéthylamine ou la pyridine, par exemple dans un solvant tel que le dichlorométhane ou l'acétate d'éthyle, par exemple à une température comprise entre 0 et 20°C. Le composé N-protégé de formule (VIII) sous forme de tertbutoxycarbonyle est ensuite déprotégé en milieu acide avec par exemple de l'acide chlorhydrique ou de l'acide trifluoroacétique) par exemple à température ambiante pour conduire aux composés de formule (III).

Les composés de formule (IV) peuvent être préparés selon le schéma 4 qui est une forme particulière du schéma 1 :

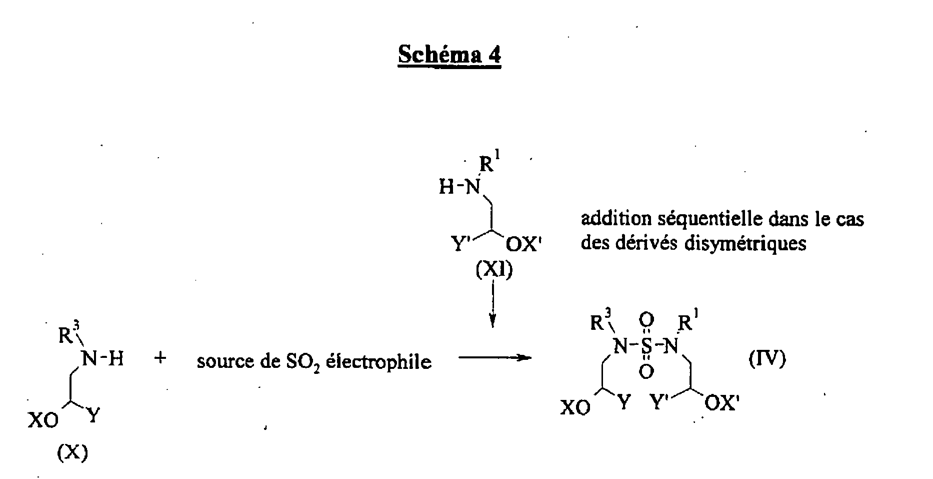

Les amines (ou diamines) de formules respectives (X) et (XI) choisies pour conduire aux composés de formule (IV) sont mises en réaction (de manière séquentielle pour les composés disymétriques) avec une source électrophile de SO₂, telle que le sulfamide H₂NSO₂NH₂, le chlorure de sulfuryle SO₂Cl₂, ou la sulfonyl-bis 2-oxazolidine dans un solvant approprié (par exemple respectivement l'acétonitrile, la pyridine, le diméthylformamide pour le sulfamide ou par exemple le dichlorométhane, le tétrahydrofurane pour le chlorure de sulfuryle) par exemple à une température variant de 0 à 140 °C en présence ou non d'une base comme par exemple le diazabicycloundécène, la triéthylamine ou la pyridine.

Le tableau ci-après rassemble les composés que l'on peut utiliser de façon préférée dans le cadre de la présente invention.

**Tableau 1**

| N° | Nom chimique | Formule |
|---|---|---|
| 1 | Sulfamide | 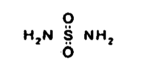 |
| 2 | N-(2-hydroxyéthyl)sulfamide | 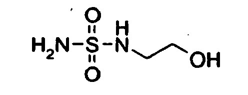 |
| 3 | N-(3-hydroxypropyl)sulfamide | 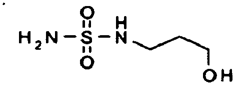 |
| 4 | N-(2,3-dihydroxypropyl)sulfamide | 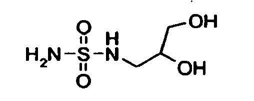 |
| 5 | N-(4-hydroxybutyl)sulfamide | 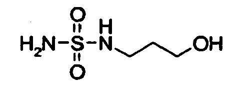 |
| 6 | N-(2,3,4,5,6,pentahydroxyhexyl)sulfamide | 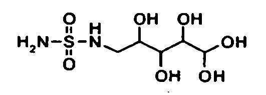 |
| 7 | N-Methyl-N-(2,3,4,5,6,pentahydroxyhexyl)sulfamide | 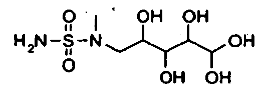 |
| 8 | N-[triméthylsilylméthyl]sulfamide | 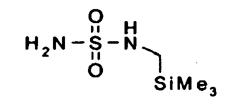 |
| 9 | N-[bis(triméthylsilyl)méthyl]sulfamide | 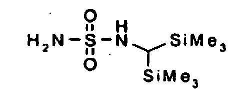 |
| 10 | N, N" bis (hydroxyéthyl)sulfamide | 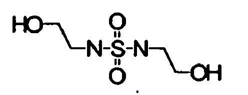 |
| 11 | N, N" bis(hydroxypropyl)sulfamide | 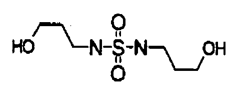 |
| 12 | N, N" bis(hydroxybutyl)sulfamide | 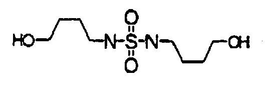 |
| 13 | N, N" bis (2,3-dihydroxypropyl)sulfaxnide | 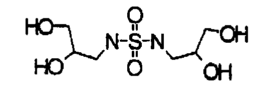 |
| 14 | N,N"-bis[(2,2-dimethyl-1,3-dioxolan-4-yl)methyl] sulfamide | 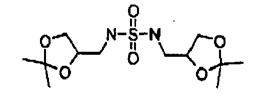 |
| 15 | N-[bis(triméthylsilyl)méthyl]-N"-(2-hydroxyéthyl)sulfamide | 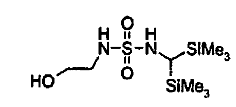 |
| 16 | N-[(triméthylsilyl)méthyl]-N"-(2-hydroxyéthyl)sulfamide | 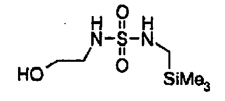 |
| 17 | 2-[(aminosulfonyl)amino]éthyl sulfamate | 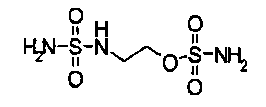 |
| 18 | 3-[(aminosulfonyl)amino]propyl sulfamate | 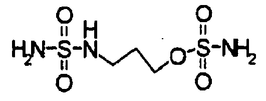 |
| 19 | (+/-) 1,2,7-thiadiazepane-4,5-diol 1,1-dioxyde | 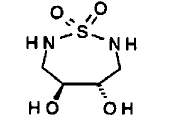 |

### Les formulations cosmétiques

La présente invention concerne une composition cosmétique comprenant un composé de formule (I) tel que décrit ci-dessus, en particulier à l'exclusion du N,N'-bis(2-hydroxyéthyl)sulfamide, dans un milieu physiologiquement acceptable, notamment à un pH non alcalin.

La présente invention a par ailleurs pour objet une composition cosmétique comprenant, dans un milieu physiologiquement acceptable, notamment à un pH non alcalin, un composé de formule (I) tel que décrit ci-dessus et comprenant en outre au moins un additif choisi parmi une huile, un corps gras, un gélifiant, une charge, un filtre UV, un absorbeur d'odeur ou une matière colorante.

Ces compositions cosmétiques dans lesquelles les composés (I), (II), (III) ou (IV) peuvent être mis en oeuvre sont utiles pour le soin non thérapeutique et/ou le maquillage de la peau. Elles sont en particulier utiles pour hydrater la peau.

Elles peuvent montrer leur efficacité à titre de traitement non thérapeutique d'entretien de la peau, à savoir à titre préventif. Elles peuvent également être utilisées à titre de traitement non thérapeutique de la peau après une manifestation de troubles de l'hydratation de la peau.

Dans ce deuxième cas, cette manifestation de troubles de l'hydratation de la peau est de préférence indépendante d'une irritation provoquée par la mise en contact de la peau avec un agent de blanchiment, notamment chloré, par exemple à base d'hypochlorite.

En outre, lesdites compositions cosmétiques ne sont de préférence pas utilisées à des fins d'hygiène, en particulier, de préférence, elles ne contiennent pas d'agent détergent.

Enfin, les compositions cosmétiques de l'invention sont de préférence formulées dans des conditions non alcalines, de façon encore plus préférée à un pH proche de celui de la peau, par exemple à un pH compris entre 5 et 6.

Les composés de formule (I), (II), (III) ou (IV) peuvent être présents dans les compositions cosmétiques dans des teneurs allant de 0,01 à 20 %, de préférence de 0,01 à 15 % et de façon encore plus préférée de 0,1 à 10 % en poids par rapport au poids total de la composition cosmétique.

Les compositions utilisées selon l'invention contiennent un milieu physiologiquement acceptable, c'est-à-dire compatible avec les tissus cutanés tels que la peau et le cuir chevelu. Ce milieu physiologiquement acceptable peut être plus particulièrement constitué d'eau et éventuellement d'un solvant organique physiologiquement acceptable choisi par exemple parmi les alcools inférieurs comportant de 1 à 8 atomes de carbone et en particulier de 1 à 6 atomes de carbone, comme l'éthanol, l'isopropanol, le propanol, le butanol ; les polyéthylène glycols ayant de 6 à 80 unités oxyde d'éthylène et les polyols comme le propylène glycol, l'isoprène glycol, le butylène glycol, la glycérine et le sorbitol.

Les compositions selon l'invention peuvent se présenter sous toutes les formes galéniques classiquement utilisées pour une application topique et notamment sous forme de solutions aqueuses, hydroalcooliques, d'émulsions huile-dans-eau (H/E) ou eau-dans-huile (E/H) ou multiple (triple : E/H/E ou H/E/H), de gels aqueux, ou de dispersions d'une phase grasse dans une phase aqueuse à l'aide de sphérules, ces sphérules pouvant être des nanoparticules polymériques telles que les nanosphères et les nanocapsules ou des vésicules lipidiques de type ionique et/ou non ionique (liposomes, niosomes, oléosomes). Ces compositions sont préparées selon les méthodes usuelles.

En outre, les compositions utilisées selon l'invention peuvent être plus ou moins fluides et avoir l'aspect d'une crème blanche ou colorée, d'une pommade, d'un lait, d'une lotion, d'un sérum, d'une pâte ou d'une mousse. Elles peuvent être éventuellement appliquées sur la peau sous forme d'aérosol. Elles peuvent aussi se présenter sous forme solide, et par exemple sous forme de stick.

Quand la composition utilisée selon l'invention comporte une phase huileuse, celle-ci contient de préférence au moins une huile. Elle peut contenir en outre d'autres corps gras.

Comme huiles utilisables dans la composition de l'invention, on peut citer par exemple :
- les huiles hydrocarbonées d'origine animale, telles que le perhydrosqualéne ;
- les huiles hydrocarbonées d'origine végétale, telles que les triglycérides liquides d'acides gras comportant de 4 à 10 atomes de carbone comme les triglycérides des acides heptanoïque ou octanoïque ou encore, par exemple les huiles de tournesol, de mais, de soja, de courge, de pépins de raisin, de sésame, de noisette, d'abricot, de macadamia, d'arara, de ricin, d'avocat, les triglycérides des acides caprylique/caprique comme ceux vendus par la société Stearineries Dubois ou ceux vendus sous les dénominations « Miglyol 810 », « 812 » et «818» par la société Dynamit Nobel, l'huile de jojoba, l'huile de beurre de karité;
- les esters et les éthers de synthèse, notamment -d'acides gras, comme les huiles de formules R₁COOR₂ et R₁OR₂ dans laquelle R₁ représente le reste d'un acide gras comportant de 8 à 29 atomes de carbone, et R₂ représente une chaîne hydrocarbonée, ramifiée ou non, contenant de 3 à 30 atomes de carbone, comme par exemple l'huile de Purcellin, l'isononanoate d'isononyle, le myristate d'isopropyle, le palmitate d'éthyl-2-hexyle, le stéarate d'octyl-2-dodécyle, l'érucate d'octyl-2-dodécyle, - l'isostéarate d'isostéaryle ; les esters hydroxylés comme l'isostéaryl lactate, l'octylhydroxystéarate, l'hydroxystéarate d'octyldodécyle, le diisostéaryl-malate, le citrate de triisocétyle ; les heptanoates, octanoates, décanoates d'alcools gras ; les esters de polyol, comme le dioctanoate de propylène glycol, le diheptanoate de néopentylglycol et le diisononanoate de diéthylèneglycol ; et les esters du pentaérythritol comme le tétraisostéarate de pentaérythrityle ;
- les hydrocarbures linéaires ou ramifiés, d'origine minérale ou synthétique, tels que les huiles de paraffine, volatiles ou non, et leurs dérivés, la vaseline, les polydécènes, le polyisobutène hydrogéné tel que l'huile de parléam ;
- les alcools gras ayant de 8 à 26 atomes de carbone, comme l'alcool cétylique, l'alcool stéarylique et leur mélange (alcool cétylstéarylique), l'octyldodécanol, le 2-butyloctanol, le 2-hexyldécanol, le 2-undécylpentadécanol, l'alcool oléique ou l'alcool linoléique ;
- les huiles fluorées partiellement hydrocarbonées et/ou siliconées comme celles décrites dans le document JP-A-2-295912 ;
- les huiles de silicone comme les polyméthylsiloxanes (PDMS) volatiles ou non à chaîne siliconée linéaire ou cyclique, liquides ou pâteux à température ambiante, notamment les cyclopolydiméthylsiloxanes (cyclométhicones) telles que la cyclohexasiloxane ; les polydiméthylsiloxanes comportant des groupements alkyle, alcoxy ou phényle, pendant ou en bout de chaîne siliconée, groupements ayant de 2 à 24 atomes de carbone ; les silicones phénylées comme les phényltriméthicones, les phényldiméthicones, les phényltriméthylsiloxydiphényl-siloxanes, les diphényldiméthicones, les diphénylméthyldiphényl trisiloxanes, les 2-phényléthyltriméthylsiloxysilicates, et les polyméthylphénylsiloxanes ;
- leurs mélanges.

On entend par « huile hydrocarbonée » dans la liste des huiles citées ci-dessus, toute huile comportant majoritairement des atomes de carbone et d'hydrogène, et éventuellement des groupements ester, éther, fluoré, acide carboxylique et/ou alcool.

Les autres corps gras pouvant être présents dans la phase huileuse sont par exemple les acides gras comportant de 8 à 30 atomes de carbone, comme l'acide stéarique, l'acide laurique, l'acide palmitique et l'acide oléique ; les cires comme la lanoline, la cire d'abeille, la cire de Carnauba ou de Candellila, les cires de paraffine, de lignite ou les cires microcristallines, la cérésine ou l'ozokérite, les cires synthétiques comme les cires de polyéthylène, les cires de Fischer-Tropsch ; les résines de silicone telles que la trifluorométhyl-C1-4-alkyldimethicone et la trifluoropropyldimethicone ; et les élastomères de silicone comme les produits commercialisés sous les dénominations « KSG » par la société Shin-Etsu, sous les dénominations « Trefil », « BY29 » ou « EPSX » par la société Dow Corning ou sous les dénominations « Gransil » par la société Grant Industries.

Ces corps gras peuvent être choisis de manière variée par l'homme du métier afin de préparer une composition ayant les propriétés, par exemple de consistance ou de texture, souhaitées.

Selon un mode particulier de réalisation de l'invention, la composition selon l'invention est une émulsion eau-dans-huile (E/H) ou huile-dans-eau (H/E). La proportion de la phase huileuse de l'émulsion peut aller de 5 à 80 % en poids, et de préférence de 5 à 50 % en poids par rapport au poids total de la composition.

Les émulsions contiennent généralement au moins un émulsionnant choisi parmi les émulsionnants amphotères, anioniques, cationiques ou non ioniques, utilisés seuls ou en mélange, et éventuellement un co-émulsionnant. Les émulsionnants sont choisis de manière appropriée suivant l'émulsion à obtenir (E/H ou H/E). L'émulsionnant et le co-émulsionnant sont généralement présents dans la composition, en une proportion allant de 0,3 à 30 % en poids, et de préférence de 0,5 à 20 % en poids par rapport au poids total de la composition.

Pour les émulsions E/H, on peut citer par exemple comme émulsionnants les dimethicone copolyols tels que le mélange de cyclomethicone et de dimethicone copolyol, vendu sous la dénomination « DC 5225 C » par la société Dow Coming, et les alkyldimethicone copolyols tels que le Laurylmethicone copolyol vendu sous la dénomination « Dow Corning 5200 Formulation Aid » par la société Dow Corning et le Cetyl dimethicone copolyol vendu sous la dénomination «Abil EM 90^{®} » par la société Goldschmidt. On peut aussi utiliser comme tensioactif d'émulsions E/H, un organopolysiloxane solide élastomère réticulé comportant au moins un groupement oxyalkyléné, tel que ceux obtenus selon le mode opératoire des exemples 3, 4 et 8 du document US-A-5,412,004 et des exemples du document US-A-5,811,487, notamment le produit de l'exemple 3 (exemple de synthèse) du brevet US-A-5,412,004. et tel que celui commercialisé sous la référence KSG 21 par la société Shin Etsu.

Pour les émulsions H/E, on peut citer par exemple comme émulsionnants, les émulsionnants non ioniques tels que les esters d'acides gras et de glycérol oxyalkylénés (plus particulièrement polyoxyéthylénés) ; les esters d'acides gras et de sorbitan oxyalkylénés ; les esters d'acides gras oxyalkylénés (oxyéthylénés et/ou oxypropylénés) ; les éthers d'alcools gras oxyalkylénés (oxyéthylénés et/ou oxypropylénés) ; les esters de sucres comme le stéarate de sucrose ; et leurs mélanges tels que le mélange de stéarate de glycéryle et de stéarate de PEG-40.

De façon connue, la composition cosmétique ou dermatologique de l'invention peut contenir également des adjuvants habituels dans le domaine cosmétique ou dermatologique, tels que les gélifiants, les polymères filmogènes, les conservateurs, les solvants, les parfums, les charges, les filtres UV, les bactéricides, les absorbeurs d'odeur, les matières colorantes, les extraits végétaux et les sels. Les quantités de ces différents adjuvants sont celles classiquement utilisées dans le domaine considéré, et par exemple de 0,01 à 20 % du poids total de la composition. Ces adjuvants, selon leur nature, peuvent être introduits dans la phase grasse et/ou dans la phase aqueuse.

Les composés de formule (I), (II), (III) et (FV) peuvent être associés entre eux, ou à d'autres agents d'hydratation de la peau non conforme à la formule (I) et/ou à au moins un autre actif cosmétique.

Au titre d'un tel actif cosmétique additionnel, on peut notamment citer les actifs agissant sur la fonction barrière de la peau, les actifs favorisant l'hydratation de la peau et les agents desquamants.

Par « agent desquamant », on entend tout composé capable d'agir :
- soit directement sur la desquamation en favorisant l'exfoliation, tel que les β-hydroxyacides, en particulier l'acide salicylique et ses dérivés (dont l'acide n-octanoyl 5-salicylique) ; les α-hydroxyacides, tels que les acides glycolique, citrique, lactique, tartrique, malique ou mandélique ; l'urée ; l'acide gentisique ; les oligofucoses ; l'acide cinnamique ; l'extrait de Saphora japonica ; le resvératrol ;
- soit sur les enzymes impliquées dans la desquamation ou la dégradation des cornéodesmosomes, telles que les glycosidases, la stratum corneum chymotryptic enzym (SCCE) voire d'autres protéases (trypsine, chymotrypsine-like). On peut citer les agents chélatant des sels minéraux : I'FDTA ; l'acide N-acyl-N,N',N' éthylène diaminetriacétique ; les composés aminosulfoniques et en particulier l'acide N-(2-hydroxyéthyl)pipérazine-N'-2-éthane sulfonique (HEPES) ; les dérivés de l'acide 2-oxothiazolidine-4-carboxylique (procystéine) ; les dérivés d'acides alpha aminés de type glycine (tels que décrits dans EP-0 852 949, ainsi que le méthyl glycine diacétate de sodium commercialisé par BASF sous la dénomination commerciale « TRILON M ») ; le miel ; les dérivés de sucre tels que l'O-octanoyl-6-D-maltose et la N-acétyl glucosamine.

Parmi les actifs agissant sur la fonction barrière de la peau, ou favorisant l'hydratation de la peau, on peut citer :
- soit les composés agissant sur la fonction barrière, en vue de maintenir l'hydratation du stratum corneum, ou les composés occlusifs, en particulier les céramides, les composés à base sphingoïde, les lécithines, les glycosphingolipides, les phospholipides, le cholestérol et ses dérivés, les phytostérols (stigmastérol, β-sitostérol, campestérol), les acides gras essentiels, le 1-2 diacylglycérol, la 4-chromanone, les triterpènes pentacycliques tels que l'acide ursolique, la vaseline et la lanoline;
- soit les composés augmentant directement la teneur en eau du stratum corneum, tel que le thréalose et ses dérivés, l'acide hyaluronique et ses dérivés, le glycérol, le pentanediol, le pidolate de sodium, la sérine, le xylitol, le lactate de sodium, le polyacrylate de glycérol, l'ectoïne et ses dérivés, le chitosane, les oligo- et polysaccharides, les carbonates cycliques, l'acide N-lauroyl pyrrolidone carboxylique, et la N-a-benzoyl-L-arginine ;
- soit les composés activant les glandes sébacées tel que les dérivés stéroïdiens (dont la DHEA) et la vitamine D et ses dérivés.

La composition peut être présentée sous forme de produit de soins non thérapeutiques et/ou de maquillage, et également de baume pour les lèvres.

L'invention a enfin pour objet un procédé de traitement cosmétique pour le soin non thérapeutique et/ou pour le maquillage de la peau caractérisé en ce qu'il comprend l'application sur la peau d'au moins une composition cosmétique selon la présente invention comprenant au moins un composé de formule (I), (II), (III) ou (IV) tel que défini ci-dessus ou un de leurs mélanges en toutes proportions.

Parmi les applications de type maquillage que le procédé de traitement cosmétique permet d'envisager, on peut notamment citer les fonds de teint, les fards à joue, les fards à paupière, les anti-cernes et le maquillage du corps.

L'invention concerne en dernier lieu l'utilisation d'un composé de formule (I), (II), (III), (IV) tel que défini ci-dessus ou d'un de leurs mélanges pour la préparation d'une composition dermatologique destinée à l'hydratation de la peau et plus particulièrement au traitement de la sécheresse cutanée ou au traitement des peaux sèches.

Les exemples ci-après illustrent l'invention sans toutefois en limiter la portée.

Les analyses élémentaires et les spectres RMN confirment les structures des produits obtenus.

Les numéros donnés entre parenthèses dans les titres des exemples correspondent à ceux du tableau 1 donné ci-dessus.

### Exemple 1 : Synthèse du N-,N"-(2,3-dihydroxypropyl)sulfamide (composé N° 13)

A une solution de 1,55 ml de dioxolane méthamine dans 25 ml de dichlorométhane et 1,84 ml de triéthylamine est ajouté goutte à goutte à 0 °C, 482 µl de chlorure de sulfuryle. Après addition, le mélange réactionnel est ramené lentement à 20 °C. Le mélange réactionnel dilué avec du dichlorométhane et lavé à l'eau puis avec une solution saturée de chlorure de sodium. La phase organique est séchée sur sulfate de sodium avant d'être concentrée sous pression réduite pour conduire à 1,6 g d'un solide blanc identifié comme le N,N'-bis[(2,2-dimethyl-1,3-dioxolan-4-yl)methyl]sulfamide.

Ce composé, le N,N"-bis[(2,2-dimethyl-1,3-dioxolan-4-yl)methyl]sulfamide, est ensuite placé en suspension dans l'eau en présence de résine Dowex^{®} 50WX8 acide vendue par Aldrich. Le milieu réactionnel se solubilise rapidement et l'avancement de la réaction est suivi par chromatographie sur couche mince sur silice. Une fois la réaction terminée, le mélange est filtré et le filtrat est évaporé sous vide pour conduire à une huile vitrifiée reprise dans un mélange d'éther et de méthanol pour conduire à 1,16 g d'un solide blanc récupéré par filtration et identifié comme le N-,N"-(2,3-dihydroxypropyl)sulfamide.

Point de fusion : 80-81 °C (éther/méthanol).

| **Analyse Elémentaire :** | | | | | |
|---|---|---|---|---|---|
| % | C | H | N | O | S |
| Calculé | 29,5 | 6,6 | 11,5 | 39,5 | 13,1 |
| Expérimentale | 29,6 | 6,6 | 11,3 | 39,2 | 13,1 |

### Exemple 2: Synthèse du 2-[(aminosulfonyl)amino]éthyl sulfamate (composé N° 17)

A 4,35 ml de chlorosufonylisocyanate dans 25 ml de dichlorométhane est ajouté goutte à goutte à 0 °C, une solution de 4,78 ml de tertbutanol dans 25 ml de dichlorométhane. Le milieu réactionnel est agité 10 minutes à 20 °C après addition avant d'être ajouté goutte à goutte à une solution de 3,01 ml d'éthanolamine, 7,66 ml de triéthylamine dans 60 ml de dichlorométhane à 0 °C. Après remontée en température à 20 °C et agitation pendant une nuit, le mélange réactionnel est dilué avec du dichlorométhane et lavé par 3 fois avec de l'acide chlorhydrique dilué. La phase organique est ensuite lavée par 3 fois avec de l'eau puis est séchée sur sulfate de sodium avant d'être concentrée sous pression réduite pour conduire à 5 g d'un solide blanc identifié comme le 2-({[(tert-butoxycarbonyl)amino]sulfonyl}amino)ethyl tert-butoxycarbonylsulfamate utilisable tel quel.

A 1,5 g de 2-({[(tert-butoxycarbonyl)amino]sulfonyl}amino)ethyl tert-butoxycarbonylsulfamate dans 30 ml de dichlorométhane est ajouté 30 ml d'un mélange 1/1 acide trifluoroacétique / dichlorométhane. Après agitation 4 heures à 20 °C, le mélange réactionnel est concentré sous pression réduite et repris 3 fois à l'éther et réévaporé. Le solide blanc est repris par du dichlorométhane et filtré sous vide pour conduire à 700 mg du produit désiré : le 2-[(aminosulfonyl)amino]éthyl sulfamate.

Point de fusion : 78-82 °C (éther/dichlorométhane).

| **Analyse Elémentaire :** | | | | | |
|---|---|---|---|---|---|
| % | C | H | N | O | S |
| Calculé | 11 | 4.1 | 19.2 | 36.5 | 29.3 |
| Expérimentale | 11.7 | 4.2 | 18.6 | 35.1 | 29.4 |

### Exemple 3 : Synthèse du (+/-) 1,2,7-thiadiazépane-4,5-diol 1,1-dioxide (composé N° 19)

A 1,5g de (+/-) 4,5-di(aminomethyl)-2,2-diméthyl dioxolane dans 30 ml de diméthylformamide sont ajoutés 1,08 g de sulfamide et 301 µl de diazabicycloundecène. Le mélange réactionnel est alors chauffé à 140°C pendant plusieurs heures jusqu'à transformation complète. Après évaporation du diméthylformamide, le brut réactionnel est chromatographié sur colonne de silice pour conduire à 1,5 g d'un solide orange. Ce solide est alors repris dans du pentane pour conduire à 1,2. g d'un solide identifié comme le 2,2-dimethythexahydro[1,3]dioxolo[4,5-d][1,2,7]thiadiaxepine 6,6-dioxide.

A 500 mg de 2,2-diméthylhexahydro[1,3]dioxolo[4,5-d][1,2,7]thiadiazepine 6,6-dioxide mis en suspension dans 10 ml d'eau est ajouté de la résine Dowex^{®} 50WX8 acide, vendu par Aldrich et 2 ml de tétrahydrofuranne. Le milieu réactionnel est agité à température ambiante pendant une nuit pour conduire après filtration de la résine et évaporation de l'eau à une pâte vitrifiée, qui reprise dans l'éther et le méthanol, conduit à 380 mg d'un solide jaune pale identifié comme le (+/-) 1,2,7-thiadiazépane-4,5-diol 1,1-dioxide.

Point de fusion : 188-192 °C (éther/méthanol)

| **Analyse Elémentaire :** | | | | | |
|---|---|---|---|---|---|
| % | C | H | N | O | S |
| Calculé | 26.4 | 5.5 | 15.4 | 35.1 | 17.6 |
| Expérimentale | 26.5 | 5.5 | 15.3 | 34.7 | 17.6 |

### Exemple 4 : Synthèse du N-(2-hydroxyéthyl)sulfamide (composé N° 2)

A 2,17 ml de chlorosufonylisocyanate dans 10 ml de dichlorométhane est ajouté goutte à goutte à 0 °C, une solution de 2,39 ml de tertbutanol dans 10 ml de dichlorométhane. Le milieu réactionnel est agité 30 minutes à 0 °C après addition avant l'ajout goutte à goutte de 10,4 ml de triéthylamine. Après agitation pendant 30 minutes 1,58 ml d'éthanolamine sont ajoutés goutte à goutte à 0 °C.

Après remontée en température à 20 °C et agitation pendant une nuit, le mélange réactionnel est concentré sous vide puis le brut est dilué avec du dichlorométhane et de l'eau ainsi qu'avec de l'acide chlorhydrique 0,1N. La phase aqueuse est alors acidifiée à l'aide d'acide chlorhydrique 1N puis est extraite par trois fois avec de l'acétate d'éthyle. Les phases organiques sont lavées à l'aide d'une solution saturée en chlorure de sodium puis sont séchées sur sulfate de sodium. Après évaporation, 2,5 g d'un solide blanc sont obtenus, séchés sous vide et identifié comme le N-(tert-butoxycarbonyl)-N"-(2-hydroxyéthyl)sulfamide.

1 g de N-(tert-butoxycarbonyl)-N"-(2-hydroxyéthyl)sulfamide est placé en suspension dans 10 ml d'acide chlorhydrique 1 N sous agitation à température ambiante. Après une nuit la réaction suivie par chromatographie sur couche mince est terminée. Le milieu réactionnel est concentré sous vide, puis coévaporé plusieurs fois avec de l'éthanol pour conduire à 520 mg d'une huile incolore identifiée comme le N-(2-hydroxyéthyl)sulfamide.

| **Analyse Elémentaire:** | | | | |
|---|---|---|---|---|
| % | C | H | O | S |
| Calculé | 17,1 | 5,8 | 34,2 | 22,9 |
| Expérimentale | 17,06 | 5,4 | 35 | 23 |

### Exemple 5 : Synthèse du N-(2,3-dihydroxypropyl)sulfamide (composé N° 4)

Mode opératoire identique à celui du N-(2-hydroxyéthyl)sulfamide de l'exemple 4 avec comme amine la 2,3 dihydroxy propylamine qui permet de conduire au composé : N-(tert-butoxycarbonyl)-N"-(2,3-dihydroxypropyl)sulfamide qui est ensuite déprotégé par de l'acide chlorhydrique comme pour le composé de l'exemple 5 pour conduire à une huile incolore identifiée comme le N-(2,3-dihydroxypropyl)sulfamide.

| **Analyse Elémentaire :** présence de 0,75 H₂O par mole | | | | | |
|---|---|---|---|---|---|
| % | C | H | N | O | S |
| Calculé | 21,1 | 5,9 | 16,4 | 37,6 | 18,8 |
| Calculé avec 0.75 H₂O | 19.6 | 6.2 | 15.2 | 41.4 | 17.4 |
| Expérimentale | 19,9 | 5,8 | 14,8 | 38,3 | 17,3 |

### Exemple 6 : Synthèse du N-[bis(trimethylsilyl)methyl]sulfamide (composé N°9)

Le mode opératoire est identique à celui du composé de l'exemple 4 avec comme amine, la bis(triméthylsilyl)méthylamine pour conduire au composé protégé : N-(tert-butoxycarbonyl)-N"-(bis(trimethylsilyl)methyl)sulfamide:

Le procédé de déprotection s'effectue par contre différemment. A une solution 30 ml de dichlorométhane et 30 ml d'acide trifluoroacétique est ajouté à 0 °C, 2 grammes de N-(tert-butoxycarbonyl)-N"-(bis(trimethylsilyl)methyl)sulfamide. L'avancement de la réaction est suivi par chromatographie sur couche mince. Après une nuit, le milieu réactionnel est concentré sous vide, repris et coévaporé à l'éther diéthylique et au dichlorométhane. La pâte obtenue est ensuite purifiée sur colonne de silice pour conduire à 500 mg d'un solide blanc identifié comme le N-[bis(trimethylsilyl)methyl]sulfamide.

Point de fusion: 76-77.5 °C (dichlorométhane).

| **Analyse Elémentaire** : | | | | |
|---|---|---|---|---|
| % | C | H | N | S |
| Calculé | 33 | 8,7 | 11 | 12,6 |
| Expérimentale | 33 | 8,5 | 11,1 | 11,9 |

### Exemple 7: Synthèse du N-[bis(triméthylsilyl)methyl]-N"-(2-hydroxyethyl)sulfamide (composé N° 15)

A 2,18 ml de chlorosufonylisocyanate dans 10 ml de dichlorométhane est ajouté goutte à goutte à 0 °C, une solution de 1,77 ml de bromo éthanol dans 10 ml de dichlorométhane. Le milieu réactionnel est agité 10 minutes à 0 °C après addition avant d'être ajouté goutte à goutte à une solution de 4,38 grammes de Bis(trimethylsilyl)méthylamine, 3,51 ml de triéthylamine dans 40 ml de dichlorométhane à 0 °C. Après remontée en température à 20 °C et agitation pendant 4 heures, le mélange réactionnel est refroidi à 0 °C avant ajout de 10,5 ml de triéthylamine goutte à goutte. Le mélange réactionnel est alors agité une nuit à température ambiante. Il est ensuite dilué avec du dichlorométhane et lavé par 3 fois avec de l'acide chlorhydrique dilué. La phase organique est ensuite lavée 1 fois avec de l'eau saturée en NaCl puis est séchée sur sulfate de sodium avant d'être concentrée sous pression réduite pour conduire à un solide blanc, l'acide 2-oxo-oxazolidine-3-sulfonique (bis-triméthylsilanyl-méthyl)-amide (rendement de 65%).

A 1 g d'acide (bis-triméthylsilanyl-méthyl)-amide 2-oxo-oxazolidine-3-sulfonique placé en suspension dans 4 ml d'éthanol, est ajouté 10 ml de soude 2N à température ambiante. Le mélange réactionnel est ainsi agité pendant 2 jours à température ambiante pour transformer le produit. Le milieu réactionnel est extrait 3 fois par de l'acétate d'éthyle. La phase organique est séchée sur sulfate de sodium et concentrée sous vide pour conduire à un solide qui est ensuite lavé avec un mélange pentane / éther.

Point de fusion : 104-106 °C (pentane/éther).

| **Analyse Elémentaire :** | | | | |
|---|---|---|---|---|
| % | C | H | N | S |
| Calculé | 36,2 | 8,8 | 9,4 | 10,7 |
| Expérimentale | 35,7 | 8,66 | 9,3 | 10,79 |

### Exemple 8: Synthèse du N,N'-bis(2-hvdroxyéthyl)sulfamide (composé n°10)

17g de sulfonyle bis-oxazolidinone sont ajoutés à une solution de soude 2N (170ml d'eau pour 13.6g de NaOH) et placés sous agitation. Après un fort dégagement de CO₂, le milieu réactionnel est agité pendant 3 jours à température ambiante. Le milieu réactionnel est alors passé sur résine Dowex® 50WX8-200 (vendue par Aldrich). La résine est filtrée sur fritté et le filtrat est concentré sous vide. Le résidu est purifié par chromatographie flash sur silice (éluant AcOEt/MeOH 8/2). Les volatils sont évaporés sous vide pour conduire à une huile incolore (Rendement 87%).

| **Analyse Elémentaire :** | | | | | |
|---|---|---|---|---|---|
| % | C | H | N | O | S |
| Calculé produit pur | 26 | 6,5 | 15,2 | 34,7 | 17,4 |
| Calculé produit avec 0.5 mole d'eau | 24,9 | 6,7 | 14,5 | 37,3 | 16,6 |
| **Expérimentale** | **25** | **6,6** | **14,5** | **36,7** | **17** |

### Exemple 9 : Evaluation du potentiel d'hydratation

Deux tests ont été mis en oeuvre pour évaluer le potentiel d'hydratation des composés de l'invention qui sont formulés dans une solution aqueuse à 3 % (sauf les composés n° 9 et n° 15 évalués dans une solution de N-lauroyl sarcosinate d'isopropyle).
- le Dermomètre, mesure mécanique de l'effet plastifiant (décrit par J de Rigal, J-L. Leveque, International Journal of Cosmetic Science, 1982, 247-260),
- la Perte Insensible en Eau (PIE), évaluation des modifications de la fonction barrière du *Stratum Corneum.*

### Dermomètre

Les tests effectués ont été réalisés dans les conditions standard sur du stratum corneum dans une enceinte régulée en température et en humidité (T=30 °C et HR=75 %). Les mesures de module élastique sont réalisées sur chaque éprouvette témoin puis 2 heures et 20 heures après application du traitement. La variation relative du module permet **d'apprécier l'effet plastifiant de l'actif sur le** *Stratum **Corneum.***

### Perte Insensible en Eau (PIE)

La perte insensible en eau est un phénomène physiologique de diffusion de vapeur d'eau à travers la couche cornée. Il résulte de la présence d'un fort gradient de pression de vapeur d'eau entre les milieux intérieurs et extérieurs du corps. La PIE dépend de l'intégrité de la fonction barrière du *Stratum Corneum.* L'appareil évaluant la PIE (Evaporimeter FP1, Servomed) mesure le flux d'eau qui diffuse passivement à travers le *Stratum Corneum* (g/m²/h).

Le stratum isolé est disposé (face interne) sur un réservoir d'eau et équilibré dans une enceinte régulée à 40% d'humidité relative (T=30°C). Préalablement à la mesure, le *Stratum Corneum* est délipidé par un traitement de deux heures dans un mélange de chloroforme et de méthanol (2v/1v). Pour chaque mesure, la PIE est mesurée sur le *Stratum Corneum* avant traitement puis 2 et 20 heures après application du traitement. La variation relative de la PIE permet d'apprécier l'évolution de la propriété de barrière du *Stratum Corneum.* Les mesures sont analysées en apparié. Pour chaque produit, 8 mesures sont réalisées sur 2 lots de *Stratum Corneum.*

La technique permet de mesurer le flux d'eau qui diffuse passivement à travers une membrane de *Stratum Corneum.* Cette mesure rend compte de l'intégrité de la fonction barrière du stratum corneum. Dans le protocole que nous utilisons, le *Stratum Corneum* est délipidé par un traitement de deux heures dans un mélange de chloroforme et de méthanol (2v/1v). Les mesures sont réalisées sur le *Stratum Corneum* témoin puis 2 heures et 20 heures après application du produit. La variation relative de la PIE permet d'apprécier l'évolution de la propriété de barrière du *Stratum Corneum.*

**Tableau 2 : Mesures au Dermomètre : Variation relative du module d'élasticité du Stratum Corneum à 30 °C et 75 % d'humidité relative, à 2 h et 20 h après application de l'actif**

| **Produit** | **Moyenne ± écart-type** | |
|---|---|---|
| | | |
| | **2h** | **20h** |
| 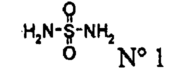 | -57±7% | -68±8% |
| 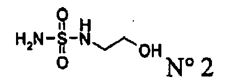 | -46+/-13% | -63+/-14% |
| 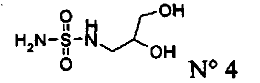 | - 41 +/- 17 % | -54+/-15% |
| 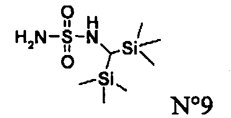 | -25+/-19% | -34+/-19% |
| 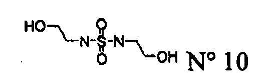 | -40 t 16 % | -49±14% |
| 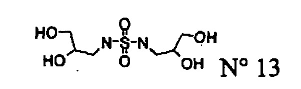 | -27±15% | -29±13% |
| 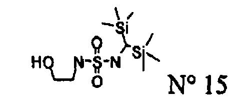 | -28±15 % | -31±24% |
| 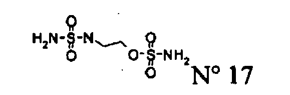 | -34±14% | -44±14% |
| 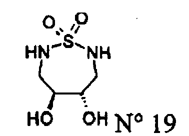 | -20±6% | -24±10% |

| Produits témoins | | |
|---|---|---|
| *stratum corneum* non traité | -4 ± 5 % | -10 ± 5 % |
| Eau pure | -5 ± 10 % | -2 ± 12 % |
| N-lauroylsarcosinate d'isopropyle¹ | -18+/-18% | -20+/-22% |
| Urée 3% dans l'eau | -72 ± 9% | -83 ± 7% |
| Glycérol 3 % dans l'eau | -41 ± 12 % | -51 ± 15 % |

| | | |
|---|---|---|
| ¹ commercialisé sous la dénomination Eldew SL205^{®} par la société Ajinomoto. | | |

La moyenne et l'écart-type ont été calculés sur 6 à 10 échantillons.

**Tableau 3. Variation relative des mesures de PIE 2 h et 20 h après application de l'actif**

| **Produit** | (Moyenne ± écart-type) | |
|---|---|---|
| | **2h** | **20h** |
| 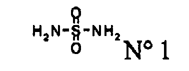 | 42,4 ± 22,6% | 37,1 ± 21,1% |
| 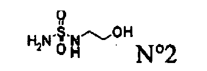 | 38 ± 18% | 37 ± 17% |
| 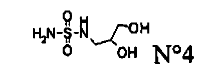 | 13 ± 11% | 1 ± 10% |
| 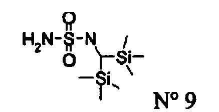 | -3 ± 5 % | - 4,5 ± 5% |
| 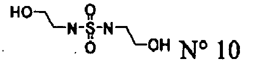 | 31,5 ± 22,1% | 21,1 ± 6,0% |
| 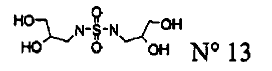 | 4,6 ± 9,4 % - | 1,0 ± 6,0% |
| 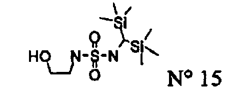 | 1 ± 8% | 1 ± 5% |
| 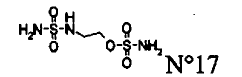 | 18 ± 15% | -0,5 ± 10% |

| Produits témoins | | |
|---|---|---|
| Eau pure | 3,5 ± 10% | -1,7 ± 5,1% |
| N-lauroylsarcosinate d'isopropyle¹ | 0,2 ± 7 % | -7 ± 4% |
| Urée 3 % dans l'eau | 64,3 ± 26,9 % | 48,3 ± 24,5 % |
| Glycérol 3 % dans l'eau | 41,2 ± 19,0 % | 33,1 ± 20,3 % |

| | | |
|---|---|---|
| ¹ commercialisé sous la dénomination Eldew SL205^{®} par la société Ajinomoto. | | |

La moyenne et l'écart-type ont été calculés sur 8 échantillons répartis sur 2 lots de *Stratum Corneum.*

Il ressort de ces tests que les composés selon l'invention possèdent des profils « hydratant » différents en plastifiant le *Stratum Corneum* et en modifiant ou non la fonction barrière.

### Exemple 10 : Formulations cosmétiques

### Exemple 10.1 : Crème de soin de la peau

| **PHASE A** | |
|---|---|
| Glyceryl stearate (et) PEG-100 stearate | 2,00 g |
| Dimyristyl tartrate (et) cetearyl alcohol (et) C12-15 pareth-7 (et) PPG-25 laureth-25 | 1,50 g |
| Cyclohexasiloxane | 5,00 g |
| Alcool Stearylique | 1,00 g |
| | |

| **PHASE B** | |
|---|---|
| Eau | QSP 100 g |
| Pentasodium ethylene diamine tetramethylene phosphate | 0,05 g |
| Ammonium Polyacryldimethyltauramide: | 0,40 g |
| Xanthan gum | 0,20 g |
| | |

| **PHASE C** | |
|---|---|
| Composé N° 4 | 3,00 à 5,00 g |
| Glycérine | 1,50 g |
| Adenosine | 0,10 g |
| Eau | 3,00 g |

### Mode opératoire

- On chauffe la phase B à environ 75 °C et on y incorpore l'Ammonium Polyacryldimethyltauramide ; on agite jusqu'à l'obtention d'un gel homogène.
- On chauffe la phase A à environ 75 °C.
- On réalise l'émulsion en incorporant la phase A dans la phase B.
- A 40-45 °C, on incorpore la phase C, et on maintient l'agitation jusqu'à refroidissement complet.

Des crèmes de soin de la peau ont également été préparées selon cette formule avec les composés N° 1, N° 3, N° 6, N° 7, N° 9, N° 13, N° 15, N° 16 et N° 19.

### Exemple 10.2 : Crème de soin de la peau

- Composé N° 2 3,0 à 6,0 %
- Monostéarate de glycérol 0,8 %
- Alcool cétylique 2,0 %
- Alcool stéarylique 5,0 %
- Stéarate de polyoxyéthylène (20 OE) 3,0 %
- Acide acrylique réticulé (CARBOPOL 941.) 0,3 %
- Triglycérides capryliques / capriques 12,0 %
- Conservateurs qs
- Eau qsp 100,0 %

Des crèmes de soin de la peau ont également été préparées selon cette formule avec les composés N° 5, N° 8, N° 10, N° 11, N° 12, N° 14, N° 17 et N° 18.

Les formulations cosmétiques exemplifiées appliquées sur la peau montrent un bon effet d'hydratation de la peau.

## Revendications

1. Utilisation cosmétique d'un composé de formule (I) 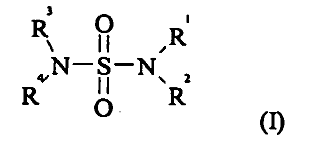 dans laquelle
- R¹, R², R³, R⁴ représentent indépendamment les uns des autres
- un atome d'hydrogène,
- un groupe (C₁-C₁₀)alkyle, de préférence un groupe (C₁-C₆)alkyle et mieux un groupe (C₁-C₄)alkyle, éventuellement substitué par 1 à 5 groupes choisis parmi -OR⁵, -NR⁶R⁷ et -SiR⁸R⁹R¹⁰, où éventuellement 1 à 3 atomes de carbone dudit groupe (C₁-C₁₀)alkyle peuvent être remplacé(s), indépendamment les uns des autres, par un hétéroatome choisi parmi un atome d'azote, un atome de soufre, un atome d'oxygène, un atome de silicium et un groupe sulfonyle (-SO₂-) incluant par exemple le groupement -O-SO₂-NR⁶R⁷, ou bien
- (R¹ et R²) et/ou (R³ et R⁴) peuvent former, avec l'azote qui les porte, un hétérocycle de 5 à 7 chaînons éventuellement substitué par 1 à 4 groupes hydroxyle et/ou un groupe -NR⁶R⁷, l'hétérocycle étant choisi notamment parmi la pyrrolidine, la pipérazine, la morpholine, l'azépane, la pyrazolidine, l'imidazolidine, l'oxazolidine, ainsi que leurs homologues partiellement insaturés par exemple dérivés du pyrrole, la pyridine ou la pyrimidine, ou bien
- (R¹ et R³) ou (R² et R⁴) peuvent former, avec le groupe -N-(SO₂)-N- qui les porte, un hétérocycle de 5 à 9 chaînons éventuellement substitué par 1 à 4 groupes hydroxyle(s) et/ou un groupe -NR⁶R⁷ et notamment choisi parmi les 1,1-dioxydes de 1,2,5- thiadiazolidine, de 1,2,6- thiadiazinane, de 1,2,7- thiadiazépane, de 1,2,8- thiadiazocane et de 1,2,9- thiadiazonane,
- R⁵, R⁶, R⁷ représentent, indépendamment les uns des autres,
- un atome d'hydrogène, ou
- un groupe (C₁-C₆)alkyle, et
- R⁵ peut encore représenter un groupe tel que -OR⁵ représente un groupe phosphate ou sulfate.
- R⁸, R⁹, R¹⁰ représentent indépendamment les uns des autres un groupe (C₁-C₆)alkyle,
ainsi que leurs sels cosmétiquement acceptables, leurs solvates et leurs isomères, pour le soin non thérapeutique de la peau.

2. Utilisation cosmétique selon la revendication 1, **caractérisée en ce que** le composé de formule (I) est tel que :
- R¹, R², R³, R⁴ représentent indépendamment les uns des autres :
- un atome d'hydrogène,
- un groupe (2,2-diméthyldioxolane)méthyle,
- un groupe (C₁-C₁₀)alkyle éventuellement substitué par 1 à 5 groupes choisis parmi -OR⁵, -NR⁶R⁷, -SiR⁸R⁹R¹⁰, ou bien
- (R¹ et R³) ou (R² et R⁴) forment ensemble, avec le groupe -N-(SO₂)-N- qui les porte, un hétérocycle de 6 à 8 chaînons choisi parmi les 1,1-dioxydes de 1,2,6-thiadiazinane, de 1,2,7-thiadiazépane, de 1,2,8-thiadiazonane, éventuellement substitués par 1 à 3 groupes hydroxyle,
- R⁵ représente :
- un atome d'hydrogène,
- un groupe (C₁-C₄)alkyle, ou
- un groupe -SO₂NRR'
- R⁶, R⁷ représentent indépendamment les uns des autres :
- un atome d'hydrogène, ou
- un groupe (C₁-C₄)alkyle,
- R⁸, R⁹, R¹⁰ représentent indépendamment les uns des autres un groupe (C₁-C₄)alkyle, et
- R et R' représentent indépendamment l'un de l'autre un atome d'hydrogène ou un groupe (C₁-C₄) alkyle,
ainsi que leurs sels cosmétiquement acceptables, leurs solvates et leurs isomères, pour le soin non thérapeutique de la peau.

3. Utilisation cosmétique selon l'une quelconque des revendications 1 ou 2, **caractérisée en ce que** le composé de formule (I) est tel que :
- R¹, R², R³, R⁴ représentent indépendamment les uns des autres :
- un atome d'hydrogène,
- un groupe (C₁-C₆)alkyle éventuellement substitué par 1 à 5 groupes hydroxyle, 1 à 2 groupes SiMe₃ ou par un groupe -O-SO₂NRR', ou bien
- (R¹ et R³) représentent un atome d'hydrogène et (R² et R⁴) forment ensemble, avec le groupe N-(SO₂)-N- qui les porte, un groupe 1,1-dioxyde de 1,2,7-thiadiazepane éventuellement substitué par 1 ou 2 groupes hydroxyle ou un groupe 1,1 -dioxyde de t,2,6-thiadiazinane éventuellement substitué par 1 ou 2 groupes hydroxyle,
- R et R' représentent indépendamment l'un de l'autre :
- un atome d'hydrogène, ou
- un groupe (C₁-C₄)alkyle,
ainsi que leurs sels cosmétiquement acceptables, leurs solvates et leurs isomères, pour le soin non thérapeutique de la peau.

4. Utilisation cosmétique selon l'une quelconque des revendications 1 à 3, d'un composé de formule (I) à titre d'agent hydratant.

5. Composé de formule générale (II) 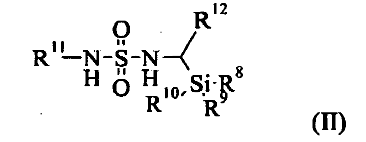 dans laquelle :
- R¹² représente un atome d'hydrogène, un groupe -SiR⁸R⁹R¹⁰, un groupe (C₁-C₉)alkyle éventuellement substitués par 1 à 5 groupes choisis parmi -OR⁵, -NR⁶R⁷, -SiR⁸R⁹R¹⁰, où éventuellement 1 à 3 atomes de carbone dudit groupe (C₁-C₉)alkyle peuvent être remplacé(s), indépendamment les uns des autres, par un hétéroatome choisi parmi un atome d'azote, un atome de soufre, un atome d'oxygène, un atome de silicium ou un groupe sulfonyle (-SO₂-),
- R¹¹ représente un atome d'hydrogène, un groupe (C₁-C₆)alkyle éventuellement substitué par 1 à 5 groupes choisis parmi -OR⁵ et -SiR⁸R⁹R¹⁰ ou éventuellement par un groupe-QSO₂-NRR',
- R⁵, R⁶, R⁷, R⁸, R⁹, R¹⁰, R et R' étant tels que définis précédemment pour les composés préférés et tout particulièrement préférés de formule (I) ainsi que leurs solvates tels que les hydrates, leurs sels et leurs isomères.

## Claims

1. Cosmetic use of a compound of formula (I) 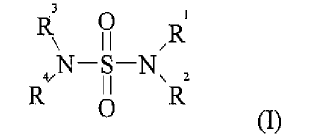 in which
- R¹, R², R³ and R⁴ represent, independently of each other
- a hydrogen atom,
- a (C₁-C₁₀)alkyl group, preferably a (C₁-C₆)alkyl group and better still a (C₁-C₄)alkyl group, optionally substituted with 1 to 5 groups chosen from -OR⁵, -NR⁶R⁷ and -SiR⁸R⁹R¹⁰, in which optionally 1 to 3 carbon atoms of the said (C₁-C₁₀)alkyl group may be replaced, independently of each other, with a hetero atom chosen from a nitrogen atom, a sulfur atom, an oxygen atom, a silicon atom and a sulfonyl group (-SO₂-) including, for example, the group -O-SO₂-NR⁶R⁷, or alternatively
- (R¹ and R²) and/or (R³ and R⁴) may form, with the nitrogen that bears them, a 5- to 7-membered heterocycle optionally substituted with 1 to 4 hydroxyl groups and/or a group -NR⁶R⁷ , the heterocycle being chosen especially from pyrrolidine, piperazine, morpholine, azepane, pyrazolidine, imidazolidine and oxazolidine, and also partially unsaturated homologues thereof, for example pyrrole, pyridine or pyrimidine derivatives, or alternatively
- (R¹ and R³) or (R² and R⁴) may form, with the group -N-(SO₂)-N- that bears them, a 5- to 9-membered heterocycle optionally substituted with 1 to 4 hydroxyl groups and/or a group -NR⁶R⁷ and chosen especially from 1,2,5-thiadiazolidine, 1,2,6-thiadiazinane, 1,2,7-thiadiazepane, 1,2,8-thiadiazocane and 1,2,9-thiadiazonane 1,1-dioxides,
- R⁵, R⁶ and R⁷ represent, independently of each other,
- a hydrogen atom, or
- a (C₁-C₆)alkyl group, and
- R⁵ may also represent a group such that -OR⁵ represents a phosphate or sulfate group,
- R⁸, R⁹ and R¹⁰ represent, independently of each other, a (C₁-C₆)alkyl group,
and also the cosmetically acceptable salts thereof, solvates thereof and isomers thereof, for non-therapeutic skincare.

2. Cosmetic use according to Claim 1, **characterized in that** the compound of formula (I) is such that:
- R¹, R², R³ and R⁴ represent, independently of each other:
- a hydrogen atom,
- a (2,2-dimethyldioxolane)methyl group,
- a (C₁-C₁₀)alkyl group optionally substituted with 1 to 5 groups chosen from -OR⁵, -NR⁶R⁷ and -SiR⁸R⁹R¹⁰, or alternatively
- (R¹ and R³) or (R² and R⁴) form, together with the -N-(SO₂)-N- group that bears them, a 6- to 8-membered heterocycle chosen from 1,2,6-thiadiazinane, 1,2,7-thiadiazepane and 1,2,8-thiadiazonane 1,1-dioxides, optionally substituted with 1 to 3 hydroxyl groups,
- R⁵ represents:
- a hydrogen atom,
- a (C₁-C₄)alkyl group, or
- a group -SO₂NRR',
- R⁶ and R⁷ represent, independently of each other:
- a hydrogen atom, or
- a (C₁-C₄)alkyl group,
- R⁸, R⁹ and R¹⁰ represent, independently of each other, a (C₁-C₄)alkyl group, and
- R and R¹ represent, independently of each other, a hydrogen atom or a (C₁-C₄) alkyl group, and also the cosmetically acceptable salts thereof, solvates thereof and isomers thereof, for non-therapeutic skincare.

3. Cosmetic use according to either of Claims 1 and 2, **characterized in that** the compound of formula (I) is such that:
- R¹, R², R³ and R⁴ represent, independently of each other:
- a hydrogen atom,
- a (C₁-C₆)alkyl group optionally substituted with 1 to 5 hydroxyl groups, 1 or 2 -SiMe₃ groups or with a group -O-SO₂NRR', or alternatively
- (R¹ and R³) represent a hydrogen atom and (R² and R⁴) together form, with the -N-(SO₂)-N- group that bears them, a 1,2,7-thiadiazepane 1,1-dioxide group optionally substituted with 1 or 2 hydroxyl groups or a 1,2,6-thiadiazinane 1,1-dioxide group optionally substituted with 1 or 2 hydroxyl groups,
- R and R' represent, independently of each other:
- a hydrogen atom, or
- a (C₁-C₄)alkyl group,
and also the cosmetically acceptable salts thereof, solvates thereof and isomers thereof, for non-therapeutic skincare.

4. Cosmetic use according to any one of Claims 1 to 3, of a compound of formula (I) as a moisturizer.

5. Compound of general formula (II) 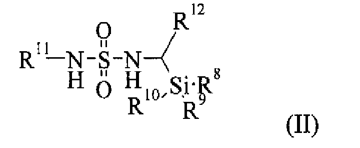 in which:
- R¹² represents a hydrogen atom, a group -SiR⁸R⁹R¹⁰, a (C₁-C₉)alkyl group optionally substituted with 1 to 5 groups chosen from -OR⁵, -NR⁶R⁷ and -SiR⁸R⁹R¹⁰, in which optionally 1 to 3 carbon atoms of the said (C₁-C₉)alkyl group may be replaced, independently of each other, with a hetero atom chosen from a nitrogen atom, a sulfur atom, an oxygen atom and a silicon atom or a sulfonyl group (-SO₂-),
- R¹¹ represents a hydrogen atom, a (C₁-C₆)alkyl group optionally substituted with 1 to 5 groups chosen from -OR⁵ and -SiR⁸R⁹R¹⁰ or optionally with a group -OSO₂-NRR',
- R⁵, R⁶, R⁷, R⁸, R⁹, R¹⁰, R and R' being as defined above for the preferred and most particularly preferred compounds of formula (I), and also the solvates thereof such as hydrates, salts thereof and isomers thereof.

6. Compound of general formula (II) according to Claim 5, **characterized in that** the compound of formula (II) is such that:
- R¹² represents a hydrogen atom, an -SiMe₃ group or a (C₁-C₆)alkyl group optionally substituted with 1 to 5 hydroxyl groups, 1 or 2 -SiMe₃ groups or with an -O-SO₂NH₂ group,
- R¹¹ represents a hydrogen atom or a (C₁-C₆)alkyl group optionally substituted with 1 or 2 groups chosen from -OR⁵ and -SiMe₃,
- R⁵, R⁸, R⁹ and R¹⁰ being as defined above for the compounds of formula (I) as defined in Claim 2 or 3.

7. Compound of general formula (II) according to Claim 5 or 6, **characterized in that** the compound of formula (II) is such that:
- R¹² represents a hydrogen atom, an -SiMe₃ group or a (C₁-C₆)alkyl group optionally substituted with a hydroxyl group,
- R¹¹ represents a hydrogen atom or a (C₁-C₆)alkyl group optionally substituted with 1 or 2 hydroxyl groups, and
- R⁸, R⁹ and R¹⁰ each represent a methyl group.

8. Compound of general formula (III) 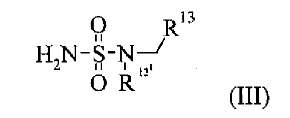 in which:
- R¹³ represents a (C₁-C₉)alkyl substituted with 1 to 5 groups chosen from -OR⁵ and a group -O-SO₂NRR',
- R^{13'} represents a hydrogen atom or a (C₁-C₄)alkyl group, and
- R⁵ represents a hydrogen atom, a (C₂-C₄) alkyl group, a group -SO₂NRR', or a group SiR⁸R⁹R¹⁰,
- R⁸, R⁹, R¹⁰, R and R' being as defined for the compounds of formula (I) according to Claim 2 or 3, and also the solvates thereof such as hydrates, salts thereof and isomers thereof.

9. Compound of general formula (III) according to Claim 8, **characterized in that** the compound of formula (III) is such that:
- R¹³ represents a (C₁-C₉)alkyl group substituted with 1 to 5 hydroxyl groups or with an -O-SO₂NH₂ group, and
- R^{13'} represents a hydrogen atom or a methyl group, and also the solvates thereof such as hydrates, salts thereof and isomers thereof.

10. Compound of general formula (IV) 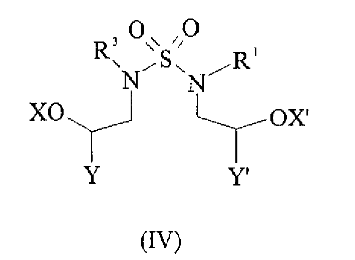
- X and X' take, independently of each other, the meaning of R⁵ as defined above for the compounds of formula (I) as defined in Claim 1 and Y and Y' represent, independently of each other, a hydrogen atom, a group -CH₂Z, in which Z represents an amine group or a group -NR⁶R⁷ or -OR⁵, with the proviso that X and X' simultaneously represent a group -CH₃, then Y and Y' are not simultaneously a hydrogen atom, or alternatively
- at least one of the groups 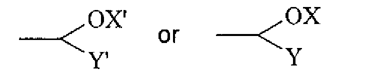 forms, independently of each other, a dimethyldioxolane group, or alternatively:
- X and X' are hydrogen atoms and Y and Y' together form a covalent bond,
- R¹, R³, R⁵, R⁶ and R⁷ take the same meaning as that defined for the compounds of formula (I) according to Claim 1, it being understood that X, X', Y and Y' do not simultaneously represent a hydrogen atom.

11. Compound of general formula (IV) according to Claim 10, **characterized in that** the compound of formula (IV) is such that:
- X and X' are hydrogen atoms and Y and Y' represent a group -CH₂OR⁵, or alternatively
- at least one of the groups: 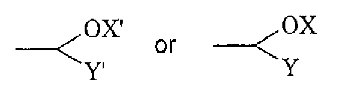 forms a dimethyldioxolane group, or alternatively
- X and X' are hydrogen atoms and Y and Y' together form a covalent bond, and
- R¹, R³ and R⁵ take the same meaning as that defined by the compounds of formula (I) according to Claim 1.

12. Cosmetic composition, **characterized in that** it contains a compound of formula (I) as defined in any one of Claims 1 to 3 and at least one additive chosen from an oil, a fatty substance, a filler, a UV-screening agent, an odour absorber and a dyestuff.

13. Cosmetic composition, **characterized in that** it contains a compound of formula (II) as defined according to any one of Claims 5 to 7.

14. Cosmetic composition, **characterized in that** it contains a compound of formula (III) as defined in Claim 8 or 9.

15. Cosmetic composition, **characterized in that** it contains a compound of formula (IV) as defined in Claim 10 or 11.

16. Cosmetic composition according to any one of Claims 12 to 15, **characterized in that** it also contains a skin moisturizer not in accordance with formula (I) according to Claim 1 and/or at least one active agent chosen from active agents that act on the barrier function of the skin, active agents that promote skin moisturization and desquamating agents.

17. Cosmetic composition according to any one of Claims 12 to 16, **characterized in that** it comprises an ingredient chosen from oils, fatty substances, emulsifiers, gelling agents, film-forming polymers, preserving agents, solvents, fragrances, fillers, UV-screening agents, bactericides, odour absorbers, dyestuffs, plant extracts and salts.

18. Cosmetic treatment process for non-therapeutic skincare and/or for making up the skin, **characterized in that** it consists in applying to the skin at least one cosmetic composition comprising a compound of formula (I), (II), (III) or (IV) as defined in any one of Claims 1 to 11, or a mixture thereof.

19. Process according to Claim 18, **characterized in that** it is a process for moisturizing the skin.

20. Use of a compound or of a mixture of compounds as defined in any one of Claims 1 to 11 for the preparation of a dermatological composition for moisturizing the skin and more particularly for treating skin dryness or for treating dry skin.

## Patentansprüche

1. Kosmetische Verwendung einer Verbindung der Formel (I) 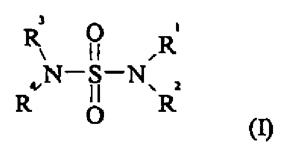 wobei
- R¹, R², R³, R⁴ unabhängig voneinander
- ein Wasserstoffatom,
- eine (C₁-C₁₀)-Alkylgruppe, vorzugsweise eine (C₁-C₆)-Alkylgruppe und stärker bevorzugt eine (C₁C₄)-Alkylgruppe, die gegebenenfalls mit 1 bis 5 Gruppen substituiert ist, ausgewählt aus -OR⁵, -NR⁶R⁷ und -SiR⁸R⁹R¹⁰, darstellen, wobei gegebenenfalls 1 bis 3 Kohlenstoffatome der (C₁-C₁₀)-Alkylgruppe unabhängig voneinander durch ein Heteroatom ersetzt sein kann (können), ausgewählt aus einem Stickstoffatom, einem Schwefelatom, einem Sauerstoffatom, einem Siliciumatom und einer Sulfonylgruppe (-SO₂-) die beispielsweise die Gruppe -O-SO₂-NR₆R₇ einschließt, oder
- (R¹ und R²) und/oder (R³ und R⁴) mit dem Stickstoffatom, das sie trägt, einen 5- bis 7-gliedrigen Heterocyclus bilden können, der gegebenenfalls mit 1 bis 4 Hydroxylgruppen und/oder einer Gruppe -NR⁶R⁷ substituiert ist, wobei der Heterocylcus insbesondere aus Pyrrolidin, Piperazin, Morpholin, Azepan, Pyrazolidin, Imidazolidin, Oxazolidin, sowie aus ihren teilweise ungesättigten Homologen, beispielsweise Derivate des Pyrrols, Pyridins oder des Pyrimidins, ausgewählt ist, oder
- (R¹ und R³) oder (R² und R⁴) mit der Gruppe -N-(SO₂)-N-, die sie trägt, einen 5- bis 9-gliedrigen Heterocyclus bilden können, der gegebenenfalls mit 1 bis 4 Hydroxylgruppe(n) und/oder einer Gruppe -NR⁶R⁷ substituiert ist und insbesondere ausgewählt ist aus 1,1-Dioxiden von 1,2,5-Thiadiazolidin, 1,2,6-Thiadiazinan, 1,2,7-Thiadiazepan, 1,2,8-Thiadiazocan und 1,2,9-Thiadiazonan,
- R⁵, R⁶, R⁷ , unabhängig voneinander,
- ein Wasserstoffatom, oder
- (C₁-C₆)-Alkylgruppe darstellen, und
- R⁵ auch eine Gruppe darstellen kann, derart dass -OR⁵ eine Phosphat- oder Sulfatgruppe darstellt,
- R⁸, R⁹, R¹⁰, unabhängig voneinander, eine (C₁-C₆)-Alkylgruppe darstellen, sowie ihrer kosmetisch verträglichen Salze, ihrer Solvate und ihrer Isomere für die nichttherapeutische Pflege der Haut.

2. Kosmetische Verwendung nach Anspruch 1, **dadurch gekennzeichnet, dass** die Verbindung der Formel (I) folgendermaßen ist:
- R¹, R², R³, R⁴ stellen unabhängig voneinander folgendes dar:
- ein Wasserstoffatom,
- eine (2,2-Dimethyldioxolan)methylgruppe,
- eine (C₁-C₁₀)-Alkylgruppe, die gegebenenfalls mit 1 bis 5 Gruppen substituiert ist, ausgewählt aus -OR⁵, -NR⁶R⁷, -SiR⁸R⁹R¹⁰, oder
- (R¹ und R³) oder (R² und R⁴) zusammen mit der Gruppe N-(SO₂)-N-, die sie trägt, einen 6 bis 8-gliedrigen Heterocyclus bilden, ausgewählt aus 1,1-Dioxiden von 1,2,6-Thiadiazinan, 1,2,7-Thiadiazepan, 1,2,8-Thiadiazonan, gegebenenfalls substituiert mit 1 bis 3 Hydroxylgruppen,
- R⁵ Folgendes darstellt:
- ein Wasserstoffatom,
- eine (C₁-C₄)-Alkylgruppe, oder
- eine -SO₂NRR'-Gruppe,
- R⁶, R⁷, unabhängig voneinander, Folgendes darstellen:
- ein Wasserstoffatom, oder
- (C₁-C₄)-Alkylgruppe,
- R⁸, R⁹, R¹⁰, unabhängig voneinander, eine (C₁-C₄)-Alkylgruppe darstellen; und
- R und R', unabhängig voneinander, ein Wasserstoffatom oder eine (C₁-C₄)-Alkylgruppe darstellen,
sowie ihre kosmetisch verträglichen Salze, ihre Solvate und ihre Isomere für die nichttherapeutische Pflege der Haut.

3. Kosmetische Verwendung nach einem der Ansprüche 1 oder 2, **dadurch gekennzeichnet, dass** die Verbindung der Formel (I) folgendermaßen ist:
- R¹, R², R³, R⁴ stellen, unabhängig voneinander, Folgendes dar:
- ein Wasserstoffatom,
- eine (C₁-C₆)-Alkylgruppe, die gegebenenfalls mit 1 bis 5 Hydroxylgruppen, 1 bis 2 SiMe₃-Gruppen oder mit einer Gruppe -O-SO₂NRR' substituiert ist, oder
- (R¹ und R³) ein Wasserstoffatom darstellen und (R² und R⁴) zusammen mit der Gruppe N-(SO₂)-N-, die sie trägt, eine 1,1-Dioxidgruppe von 1,2,7-Thiadiazepan, gegebenenfalls substituiert mit 1 oder 2 Hydroxylgruppen, oder eine 1,1-Dioxidgruppe von 1,2,6-Thiadiazinan, gegebenenfalls mit 1 oder 2 Hydroxylgruppen substituiert, darstellen,
- R und R' stellen, unabhängig voneinander, Folgendes dar:
- ein Wasserstoffatom, oder
- eine (C₁-C₄)-Alkylgruppe,
sowie ihre kosmetisch verträglichen Salze, ihre Solvate und ihre Isomere für die nichttherapeutische Pflege der Haut.

4. Kosmetische Verwendung nach einem der Ansprüche 1 bis 3 einer Verbindung der Formel (I) als Hydratationsmittel.

5. Verbindung der allgemeinen Formel (II) 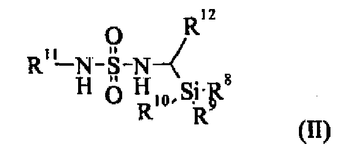 wobei
- R¹² ein Wasserstoffatom, eine Gruppe -SiR⁸R⁹R¹⁰, eine (C₁-C₉)-Alkylgruppe, die gegebenenfalls mit 1 bis 5 Gruppen substituiert ist, ausgewählt aus -OR⁵, -NR⁶R⁷, - SiR⁸R⁹R¹⁰, wobei gegebenenfalls 1 bis 3 Kohlenstoffatome der (C₁-C₉)-Alkylgruppe unabhängig voneinander durch ein Heteroatom ersetzt sein können, ausgewählt aus einem Stickstoffatom, einem Schwefelatom, einem Sauerstoffatom, einem Siliciumatom oder einer Sulfonylgruppe (-SO₂-) darstellt,
- R¹¹ ein Wasserstoffatom, eine (C₁-C₆)-Alkylgruppe, die gegebenenfalls mit 1 bis 5 Gruppen substituiert ist, ausgewählt aus -OR⁵ und -SiR⁸R⁹R10 oder gegebenenfalls einer Gruppe -OSO₂₋NRR', darstellt,
- wobei R⁵, R⁶, R⁷, R⁸, R⁹, R¹⁰, R und R' sind, wie zuvor für die bevorzugten Verbindungen und ganz besonders bevorzugt für die Formel (I) definiert, sowie ihre Solvate, wie die Hydrate, ihre Salze und ihre Isomere.

6. Verbindung der allgemeinen Formel (II) nach Anspruch 5, **dadurch gekennzeichnet, dass** die Verbindung der Formel (II) folgendermaßen ist:
- R¹² stellt ein Wasserstoffatom, eine Gruppe -SiMe₃ oder eine (C₁-C₆)-Alkylgruppe dar, die gegebenenfalls mit 1 bis 5 Hydroxylgruppe(n), 1 bis 2 Gruppen -SiMe₃ oder mit einer Gruppe -O-SO₂NH₂ substituiert sind,
- R¹¹ stellt ein Wasserstoffatom oder eine (C₁-C₆)-Alkylgruppe, die gegebenenfalls mit 1 bis 2 Gruppen substituiert ist, ausgewählt aus -OR⁵ und -SiMe₃, dar,
- wobei R⁵, R⁸, R⁹ und R¹⁰ so sind, wie zuvor für die Verbindungen der Formel (I) definiert, wie in Anspruch 2 oder 3 definiert.

7. Verbindung der allgemeinen Formel (II) nach Anspruch 5 oder 6, **dadurch gekennzeichnet, dass** die Verbindung der Formel (II) folgendermaßen ist:
- R¹² stellt ein Wasserstoffatom, eine Gruppe -SiMe₃ oder eine (C₁-C₆)-Alkylgruppe, die gegebenenfalls mit einer Hydroxylgruppe substituiert ist, dar,
R¹¹ stellt ein Wasserstoffatom oder eine (C₁-C₆)-Alkylgruppe, die gegebenenfalls mit 1 bis 2 Hydroxylgruppen substituiert ist, dar, und
- R⁸, R⁹ und R¹⁰ stellen jeweils eine Methylgruppe dar.

8. Verbindung der allgemeinen Formel (III) 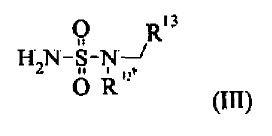 wobei:
- R¹³ ein (C₁-C₉)-Alkyl, substituiert mit 1 bis 5 Gruppen, ausgewählt aus -OR⁵ oder einer -O-SO₂NRR', darstellt,
- R¹³' ein Wasserstoffatom oder eine (C₁-C₄)-Alkylgruppe darstellt, und
- R⁵ ein Wasserstoffatom, eine (C₂-C₄)-Alkylgruppe, eine Gruppe -SO₂NRR', oder eine Gruppe SiR⁸R⁹R¹⁰ darstellt,
- wobei R⁸, R⁹, R¹⁰, R und R' so sind, wie für die Verbindung der Formel (I) nach Anspruch 2 oder 3 definiert, sowie ihre Solvate, wie die Hydrate, ihre Salze und ihre Isomere.

9. Verbindung der allgemeinen Formel (III) nach Anspruch 8, **dadurch gekennzeichnet, dass** die Verbindung der Formel (III) folgendermaßen ist:
- R¹³ stellt eine (C₁-C₉)-Alkylgruppe dar, die mit 1 bis 5 Hydroxylgruppe(n) oder mit einer Gruppe -O-SO₂NH₂ substituiert ist, und
- R¹³ stellt ein Wasserstoffatom oder eine Methylgruppe dar, sowie ihre Solvate, wie die Hydrate, ihre Salze und ihre Isomere.

10. Verbindung der allgemeinen Formel (IV) 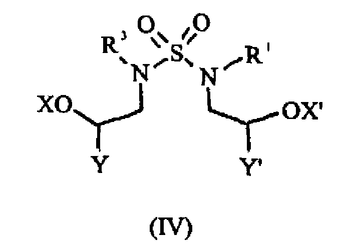
- wobei X und X', unabhängig voneinander, die Bedeutung von R⁵, wie zuvor für die Verbindungen der Formel (I), wie in Anspruch 1 definiert, definiert, einnehmen, und Y und Y¹, unabhängig voneinander, ein Wasserstoffatom, eine Gruppe -CH₂Z, wobei Z eine Amingruppe oder eine Gruppe -NR⁶R⁷ oder -OR⁵ darstellt, darstellen, mit der Maßgabe, dass, wenn X und X' gleichzeitig eine -CH₃-Gruppe darstellen, dann Y und Y' nicht gleichzeitig ein Wasserstoffatom sind, oder
- mindestens eine der Gruppen 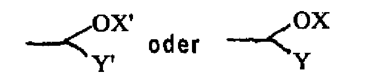 unabhängig voneinander eine Dimethyldioxolangruppe bilden, oder:
- X und X' Wasserstoffatome sind, und Y und Y' zusammen eine kovalente Bindung bilden,
- R¹, R³, R⁵, R⁶ und R⁷ die gleiche Bedeutung wie für die Verbindung der Formel (I) nach Anspruch 1 definiert einnehmen, mit der Maßgabe, dass X, X', Y und Y' nicht gleichzeitig ein Wasserstoffatom darstellen.

11. Verbindung der allgemeinen Formel (IV) nach Anspruch 10, **dadurch gekennzeichnet, dass** die Verbindung der Formel (IV) folgendermaßen ist:
- X und X' sind Wasserstoffatome und Y und Y' stellen eine Gruppe -CH₂OR⁵ dar, oder
- mindestens eine der Gruppen: 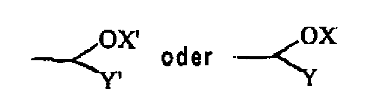 bildet eine Dimethyldioxolangruppe, oder
- X und X' sind Wasserstoffatome und Y und Y' bilden zusammen eine kovalente Bindung, und
- R¹, R³ und R⁵ nehmen die gleiche Bedeutung ein, wie durch die Verbindungen der Formel (I) nach Anspruch 1 definiert.

12. Kosmetische Zusammensetzung, **dadurch** charakterisiert, dass sie eine Verbindung der Formel (I), wie nach einem der Ansprüche 1 bis 3 definiert, und mindestens einen Hilfsstoff enthält, ausgewählt aus einem Öl, einem Fettkörper, einem Füllstoff, einem UV-Filter, einem Geruchsabsorber oder einem Farbstoff.

13. Kosmetische Zusammensetzung, **dadurch gekennzeichnet, dass** sie eine Verbindung der Formel (II), wie nach einem der Ansprüche 5 bis 7 definiert, enthält.

14. Kosmetische Zusammensetzung, **dadurch gekennzeichnet, dass** sie eine Verbindung der Formel (III), wie nach Anspruch 8 oder 9 definiert, enthält.

15. Kosmetische Zusammensetzung, **dadurch gekennzeichnet, dass** sie eine Verbindung der Formel (IV), wie nach Anspruch 10 oder 11 definiert, enthält.

16. Kosmetische Zusammensetzung nach einem der Ansprüche 12 bis 15, **dadurch gekennzeichnet, dass** sie weiterhin ein Hydratationsmittel für die Haut nicht gemäß der Formel (I) nach Anspruch 1 und/oder mindestens einen Wirkstoff, ausgewählt aus den Wirkstoffen, die auf die Barrierefunktion der Haut wirken, wobei die Wirkstoffe die Hydratation der Haut begünstigen, und aus Abschminkmitteln, enthält.

17. Kosmetische Zusammensetzung nach einem der Ansprüche 12 bis 16, **dadurch gekennzeichnet, dass** sie eine Bestandteil enthält, der ausgewählt ist aus Ölen, Fettkörpern, Emulgatoren, Geliermitteln, filmbildenden Polymeren, Konservierungsmitteln, Lösungsmitteln, Duftstoffen, Füllstoffen, UV-Filtern, Bakteriziden, Geruchsabsorbern, Farbstoffen, Pflanzenextrakten und Salzen.

18. Verfahren zur kosmetischen Behandlung zur nicht-therapeutischen Pflege und/oder zum Schminken der Haut, **dadurch gekennzeichnet, dass** es darin besteht, auf die Haut mindestens eine kosmetische Zusammensetzung aufzubringen, die eine Verbindung der Formel (I), (II), (III), (IV), wie nach einem der Ansprüche 1 bis 11 definiert, oder eines ihrer Gemische umfasst.

19. Verfahren nach Anspruch 18, **dadurch gekennzeichnet, dass** es sich um ein Hydratationsverfahren der Haut handelt.

20. Verwendung einer Verbindung oder eines Gemisches von Verbindungen, wie nach einem der Ansprüche 1 bis 11 definiert, zur Herstellung einer dermatologischen Zusammensetzung, die zur Hydratation der Haut und insbesondere zur Behandlung der Hauttrockenheit oder zur Behandlung von trockenen Häuten bestimmt ist.
